(19) Europäisches Patentamt
European Patent Office
Office européen des brevets

(11) EP 1 488 776 A1

(12) **EUROPEAN PATENT APPLICATION**
published in accordance with Art. 158(3) EPC

(43) Date of publication:
**22.12.2004 Bulletin 2004/52**

(21) Application number: **03715577.7**

(22) Date of filing: **28.03.2003**

(51) Int Cl.7: **A61K 7/06**, A61P 17/14, C07C 255/42

(86) International application number:
**PCT/JP2003/003947**

(87) International publication number:
**WO 2003/082234 (09.10.2003 Gazette 2003/41)**

(84) Designated Contracting States:
**AT BE BG CH CY CZ DE DK EE ES FI FR GB GR HU IE IT LI LU MC NL PT RO SE SI SK TR**

(30) Priority: **28.03.2002 JP 2002092377**

(71) Applicant: **Shiseido Co., Ltd.**
**Tokyo 104-8010 (JP)**

(72) Inventors:
- **HANYU, Naoto c/o Shiseido Research Center Yokohama-shi, Kanagawa 224-8558 (JP)**
- **KOBAYASHI, Koji c/o Shiseido Research Center Yokohama-shi, Kanagawa 224-8558 (JP)**
- **TAJIMA, Masahiro c/o Shiseido Research Center Yokohama-shi, Kanagawa 224-8558 (JP)**
- **ISHINO, Akihiro c/o Shiseido Research Center Yokohama-shi, Kanagawa 224-8558 (JP)**

(74) Representative: **Gudat, Axel, Dr.**
**Lippert, Stachow & Partner**
**Frankenforster Strasse 135-137**
**51427 Bergisch Gladbach (DE)**

# (54) PHENYLACETONITRILE DERIVATIVES AND HAIR TONICS AND EXTERNAL PREPARATIONS FOR SKIN CONTAINING THE DERIVATIVES

(57) A hair growth promoting composition comprising, as an effective ingredient, a phenylacetonitrile derivative or a pharmacologically acceptable salt thereof expressed by the following Formula (I):

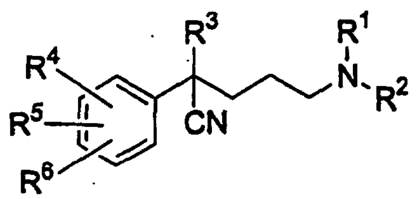

(I)

wherein each of $R^1$ and $R^2$ is hydrogen atom, a $C_{1-10}$ alkyl group, a $C_{2-10}$ alkenyl group or $C_{2-10}$ acyl group, or $NR^1R^2$ may be a heterocycle having 3-7 members; $R^3$ is a $C_{1-5}$ alkyl group; and each of $R^4$, $R^5$ and $R^6$ is hydrogen atom or a $C_{1-4}$ alkoxy group. The present invention provides a hair growth promoting composition having excellent effects on promoting hair regrowth, preventing or inhibiting hair loss, and the like in human by compounding above-specified phenylacetonitrile derivative as a effective ingredient thereto.

EP 1 488 776 A1

## Description

RELATED APPLICATIONS

This application claims the priority of Japanese Patent Application No. 2002-92377 filed on March 28, 2002, which is incorporated herein by reference.

FIELD OF THE INVENTION

The present invention relates to a phenylacetonitrile derivative, and a hair growth promoting composition and external preparation for skin using the same.

BACKGROUND OF THE INVENTION

At the present time, scalp abnormality due to activation of androgen in an organ such as hair root or sebaceous gland, lowering of blood stream toward hair follicle, excess secretion of sebum, formation of peroxide and the like has been considered as a cause of baldness or hair loss. Accordingly, for a long time, a compound or composition that can remove or reduce the above-mentioned problems has been generally included into a hair growth promoting composition (it is also called a hair growth composition, a hair regrowth promoting composition or the like), which purpose is to promote hair growth and regrowth and to prevent hair loss.

At present, compounds or crude drug extracts having various functions have been compounded to the hair growth promoting composition in various combinations. These functions include blood flow promoting action, topical stimulation, hair follicle activating action, antiandrogen action, antiseborrheic action and the like have been known. Examples of drugs having blood flow promoting action include swertia herb extract, vitamin E and its derivative, and benzyl nicotinate. Examples of drugs that promote blood circulation by topical stimulation include capsicum tincture, cantharides tincture, camphor and vanillic acid nonylamide. Examples of drugs having hair follicle activating action include hinokitiol, placental extract, photosensitizing dye, pantothenic acid and derivative thereof. Examples of drugs having antiandrogen action include estradiol and estrone. Examples of drugs having antiseborrheic action include sulfur, thioxolone and vitamin $B_6$.

In addition, salicylic acid, resorcine and the like that have corneocyte desquamating action and antibacterial action can be compounded to hair growth promoting composition for the purpose of preventing dandruff. Further, glycyrrhizic acid, menthol and the like can be compounded in order to prevent inflammation of scalp. Furthermore, amino acids, vitamins, extracts of crude drugs and the like can be compounded so as to aliment to hair follicle and activate enzyme activity.

Meanwhile, for example, *D*(*L*)-pantolactone (Unexamined Japanese Patent Publication No. Hei 8-26942), 2 (1*H*)-pyridone derivative (Unexamined Japanese Patent Publication No. Hei 8-20521), *NG*-nitro-L-arginine (Unexamined Japanese Patent Publication No. Hei 7-316023), 3-methyleneisoindolin-1-one derivative (Unexamined Japanese Patent Publication No. Hei 7-316022), indole derivative (Unexamined Japanese Patent Publication No. Hei 7-304736) are disclosed in recent patents as drugs having hair regrowth effect, hair growth effect, and hair loss protecting effect.

However, although the drugs described above are compounded to the conventional hair growth promoting compositions, they do not always exhibit sufficient effect.

SUMMARY OF THE INVENTION

The present invention has been performed in view of the foregoing problem in the prior art and its object of the present invention is to provide a compound that is excellent in hair growth promoting effect such as a hair regrowth promoting or hair growing on human hair, and a hair growth promoting composition comprising the same as an active ingredient.

As a result of diligent studies of the inventors for attaining the above-mentioned objects, it has been found that certain phenylacetonitrile derivative has a growth-stimulative effect on hair follicle cell and is useful as a hair growth promoter, thereby accomplishing the present invention.

Namely, a hair growth promoting composition in accordance with the present invention comprises, as an effective ingredient, a phenylacetonitrile derivative or a pharmacologically acceptable salt thereof expressed by the following Formula (I):

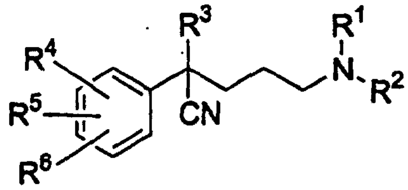

(I)

wherein

each of $R^1$ and $R^2$ is hydrogen atom, a $C_{1-10}$ alkyl group, a $C_{2-10}$ alkenyl group or $C_{2-10}$ acyl group, or $NR^1R^2$ may be a heterocycle having 3-7 members;
$R^3$ is a $C_{1-5}$ alkyl group; and
each of $R^4$, $R^5$ and $R^6$ is hydrogen atom or a $C_{1-4}$ alkoxy group.

In the hair growth promoting composition of the present invention, it is preferable that said phenylacetonitrile derivative is expressed by the following Formula (I-1):

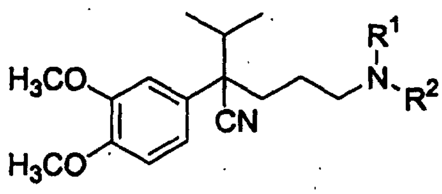

(I-1)

wherein $R^1$ and $R^2$ are as defined in said Formula (I).
Also, it is preferable that one of $R^1$ and $R^2$ is hydrogen atom or an alkyl group and the other is an alkyl group.
An external preparation for skin in accordance with the present invention comprises a phenylacetonitrile derivative or a pharmacologically acceptable salt thereof expressed by the following Formula (I):

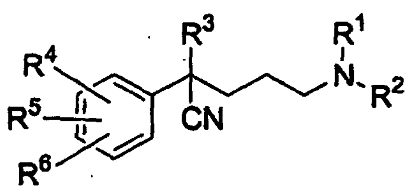

(I)

wherein

each of $R^1$ and $R^2$ is hydrogen atom, a $C_{1-10}$ alkyl group, a $C_{2-10}$ alkenyl group or $C_{2-10}$ acyl group, or $NR^1R^2$ may be a heterocycle having 3-7 members;
$R^3$ is a $C_{1-5}$ alkyl group; and
each of $R^4$, $R^5$ and $R^6$ is hydrogen atom or a $C_{1-4}$ alkoxy group.

A phenylacetonitrile derivative in accordance with the present invention is a compound or a pharmacologically acceptable salt thereof expressed by the following Formula (I-2):

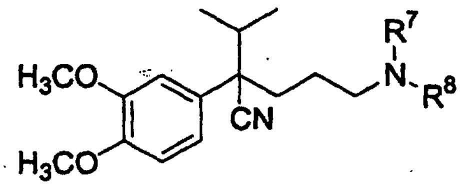

(I-2)

wherein each of $R^7$ and $R^8$ is hydrogen atom, a $C_{1-5}$ alkyl group or a $C_{2-5}$ alkenyl group, or $NR^7R^8$ may be a nonaromatic heterocycle having 3-7 members containing one nitrogen atom or an aromatic heterocycle having 5-7 members containing 1-3 nitrogen atoms, and wherein when one of $R^7$ and $R^8$ is hydrogen atom or methyl group, the other is $C_{2-5}$ alkyl group or $C_{2-5}$ alkenyl group.

BEST MODE FOR CARRYING OUT THE INVENTION

In the present invention, $R^1$ and $R^2$, which may be identical or different from each other, can be hydrogen atom, a $C_{1-10}$ alkyl group, a $C_{2-10}$ alkenyl group or a $C_{1-10}$ acyl group. Also, $NR^1R^2$ may be a heterocycle having 3-7 members. Here, in Formula (I) or (I-1), it is preferable that one of $R^1$ and $R^2$ is hydrogen atom or an alkyl group and the other is an alkyl group. More preferably, both of $R^1$ and $R^2$ are alkyl groups.

In $R^1$ and $R^2$, the $C_{1-10}$ alkyl group represents a straight or branched alkyl group having 1-10 carbon atoms, preferably having 1-5 carbon atoms. Examples thereof include methyl, ethyl, propyl, isopropyl, butyl, 1-methylpropyl, *tert*-butyl, isobutyl, pentyl, 1-methylbutyl, 2-methylbutyl, 1-ethylpropyl, isopentyl, neopentyl, hexyl, 2-ethylhexyl, heptyl, nonyl and decyl. Also, a part or the whole of the alkyl group may be cyclic. Examples of the cycloalkyl group include cyclopropyl, cyclopentyl and cyclohexyl groups.

In $R^1$ and $R^2$, the $C_{2-10}$ alkenyl group represents a straight or branched unsaturated aliphatic hydrocarbon group having 2-10 carbon atoms containing at least one double bond. Examples thereof include vinyl, allyl, metallyl, 2-pentenyl, 2-butenyl, prenyl, 3-octenyl and 4-decenyl. Also, a part or the whole of the alkenyl group may be cyclic. Examples of the cycloalkenyl group include cyclopropenyl, cyclobutenyl, cyclopentenyl and cyclohexenyl groups.

In the $R^1$ and $R^2$, the acyl group represents a carbonyl group having 1-10 carbon atoms as a total, which has hydrogen atom, an alkyl group, an aryl group or an alkenyl group. Examples thereof include acetyl, propionyl, butyryl, acryloyl, benzoyl, toluoyl and cinnamoyl.

The heterocycle formed by $NR^1R^2$ represents a saturated or unsaturated heterocycle having 3-7 members containing the nitrogen atom to which $R^1$ and $R^2$ are bonded. In addition, this heterocycle may contain one or two hetero atoms selected from nitrogen, oxygen and sulfur, respectively. Examples of the heterocycle include aziridine, azetidine, pyrrolidine, piperidine, hexamethyleneimine, homopiperazine, piperazine, morpholine, pyrrole, pyrazole and imidazole rings. Among these heterocycles, pyrrolidine, piperidine, piperazine or morpholine ring is preferable.

In Formula (I), $R^3$ represents a straight or branched alkyl group having 1-5 carbon atoms. Examples thereof include methyl, ethyl, propyl, isopropyl, butyl, 1-methylpropyl, *tert*-butyl, isobutyl,pentyl,1-methylbutyl, 2-methylbutyl, 1-ethylpropyl, isopentyl and neopentyl. Also, a part or the whole thereof may be cyclic. Examples of the cycloalkyl group include cyclopropyl and cyclopentyl groups. $R^3$ is preferably isopropyl group.

In Formula (I), each of $R^4$ to $R^6$, which may be identical or different from each other, can be hydrogen atom or a $C_{1-4}$ alkoxy group. The $C_{1-4}$ alkoxy group represents an oxy group having an alkyl group of 1-4 carbon atoms, which may be straight, branched or cyclic. Examples thereof include methoxy, ethoxy, propyloxy, *n*-butoxy and *tert*-butoxy. Preferably, it is methoxy.

In the phenylacetonitrile derivative shown by said Formula (I-2), each of $R^7$ and $R^8$, which may be identical or different from each other, represents hydrogen atom, a $C_{1-5}$ alkyl group alkyl group or a $C_{2-5}$ alkenyl group. Also, $NR^7R^8$ may be a nonaromatic heterocycle having 3-7 members containing one nitrogen atom or a aromatic heterocycle having 5-7 members containing 1-3 nitrogen atoms. However, when one of $R^7$ and $R^8$ is hydrogen atom or methyl group, the other is $C_{2-5}$ alkyl group or $C_{2-5}$ alkenyl group. In Formula (I-2), it is preferably that $R^7$ and $R^8$ are alkyl groups.

In $R^7$ and $R^8$, the $C_{1-5}$ alkyl group represents a straight or branched alkyl group having 1-5 carbon atoms. Examples thereof include methyl, ethyl, propyl, isopropyl, butyl, 1-methylpropyl, *tert*-butyl, isobutyl, pentyl, 1-methylbutyl, 2-methylbutyl, 1-ethylpropyl, isopentyl and neopentyl. Also, a part or the whole of the alkyl group may be cyclic. Examples of the cycloalkyl group include cyclopropyl and cyclopentyl groups.

In $R^7$ and $R^8$, the alkenyl group represents a straight or branched unsaturated aliphatic hydrocarbon group having 2-5 carbon atoms containing at least one double bond. Examples thereof include allyl, metallyl, 2-butenyl and

prenyl. Also, a part or the whole of the alkenyl group may be cyclic. Examples of the cycloalkenyl group include cyclopropenyl, cyclobutenyl and cyclopentenyl groups.

The nonaromatic heterocycle formed by $NR^7R^8$ represents a saturated or unsaturated nonaromatic heterocycle having 3-7 members containing the nitrogen atom to which $R^7$ and $R^8$ are bonded. In addition, this nonaromatic heterocycle may contain one or two hetero atoms selected from oxygen and sulfur, respectively. Examples of the nonaromatic heterocycle include aziridine, azetidine, pyrrolidine, piperidine, hexamethyleneimine and morpholine rings. Among them, pyrrolidine, piperidine, piperazine or morpholine ring is preferable.

The aromatic heterocycle formed by $NR^7R^8$ represents an aromatic heterocycle having 5-7 members containing the nitrogen atom to which $R^7$ and $R^8$ are bonded. In addition, this aromatic heterocycle may contain one or two nitrogen atoms. Examples of the aromatic heterocycle include pyrrole, imidazole, pyrazole and triazole rings. Among them, pyrrole, imidazole or pyrazole ring is preferable.

The hair growth promoting composition or the external preparation for skin is characterized by comprising a phenylacetonitrile derivative of said Formula (I).

Japanese Patent No. 2569088, Unexamined Japanese Patent Publication No. Sho 62-167752, Examined Japanese Patent Publication No. Hei 4-49538 and Unexamined Japanese Patent Publication No. Hei 3-291261 disclose phenylacetonitrile derivatives which have a calcium antagonism action or serotonin-bonding action and is useful as a drug for treating coronary artery disease or hypertension. Also, Unexamined Japanese Patent Publication No. 4-500067 discloses phenylacetonitrile derivatives effective for destructing anticancer drug resistance.

However, it is not disclosed in any publications mentioned above that the phenylacetonitrile derivative expressed by said Formula (I) of the present invention has a hair growth promoting effect and is applied to an external preparation for skin.

A preferable example of the phenylacetonitrile derivative (I), which is compounded in the hair growth promoting composition or the external preparation for skin of the present invention, is a compound expressed by said Formula (I-1).

Also, among phenylacetonitrile derivative (I), the compound of said Formula (I-2) has not been reported until now. Therefore, it is a novel.

The phenylacetonitrile derivative expressed by Formula (I) of the present invention may have one or more asymmetric centers. The present invention can include enantiomer, diastereomer and a mixture thereof based on the asymmetric carbon. Also, when there are the other isomers such as conformational isomers and geometrical isomers, the present invention can include them.

As long as effects of the present invention can be exhibited, the alkyl group, alkenyl group and heterocycle having 3-7 carbon atoms in said $R^1$ and $R^2$ can have at least one substituent (e.g. 1-5 substituents) at a feasible position. Examples ofthe substituent include a $C_{3-8}$ cycloalkyl, hydroxy, mercapto, cyano, carbamoyl, carboxyl, a $C_{1-4}$ alkoxycarbonyl (e.g. methoxycarbonyl, ethoxycarbonyl and the like), halogen (fluorine, chlorine, bromine, iodine), a group expressed by $-(OA^1)n-OA^2$ ,wherein $A^1$ is a $C_{1-4}$ alkylene, $A^2$ is a $C_{1-4}$ alkyl, and n is an integer of 0-3 (e.g., methoxy, ethoxy, propoxy, isopropoxy, butoxy, isobutoxy, sec-butoxy, *tert*-butoxy, ethoxyethoxy, methoxyethoxyethoxy and the like), phenoxy, halogenophenoxy (e.g., *o*-, *m*- or *p*-chlorophenoxy, *o*-, *m*- or *p*-bromophenoxy and the like), a $C_{1-4}$ alkylhthio (e.g., methylthio, ethylthio, *n*-propylthio, isopropylthio, *n*-butylthio, *tert*-butylthio, and the like), phenylthio, a $C_{1-4}$ alkylsulfinyl (e.g., methylsulfinyl, ethylsulfinyl and the like), a $C_{1-4}$ alkylsulfonyl (e.g., methylsulfonyl, ethylsulfonyl and the like), a $C_{1-10}$ haloalkyl (e.g., difluoromethyl, trifluoromethyl, trifluoroethyl, trichloroethyl and the like), formyl, a $C_{1-5}$ alkanoyl (e.g., acetyl and the like) and benzoyl.

In addition, examples of the substituent can include an unsubstituted or substituted amino, i.e., a $C_{1-6}$ alkanoylamino (e.g., acetylamino, propionylamino and the like), a $C_{1-30}$ alkylamino (e.g., methylamino, ethylamino, *n*-propylamino, isopropylamino, *n*-butylamino, isobutylamino, sec-butylamino, *tert*-butylamino, pentylamino, hexylamino, heptylamino, octylamino, nonylamino, decylamino, undecylamino, dodecylamino, tridecylamino, tetradecylamino, pentadecylamino, hexadecylamino, heptadecylamino, octadecylamino, nonadecylamino, icosylamino, henicosylamino, docosylamino, tricosylamino, tetracosylamino, pentacosylamino, hexacosylamino, heptacosylamino, octacosylamino, nonacosylamino, triacontylamino and the like, in which the alkyl group may be substituted with hydroxy group), a di-$C_{1-4}$ alkylamino (e.g., dimethylamino, diethylamino, *N*-methyl-*N*-ethylamino, *N*-methyl-*N*-propylamino and the like).

Further, examples of the substituent can include a heterocycle group having 5 or 6 members, e.g., an unsubstituted or substituted heterocycle group containing 1-4 hetero atoms selected from oxygen, sulphur and nitrogen atoms. Examples of the heterocycle include pyrrolidyl, piperidyl, morpholino, 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 2-, 4- or 5-imidazolyl, 1, 2, 3- or 1, 2, 4-triazolyl, 1H- or 2H-tetrazolyl, 2-, 3- or 4-pyridyl, 2-, 4- or 5-pyrimidyl, 3- or 4-pyridazinyl, quinolyl, imidaquinolyl and indolyl. These heterocycle group having 5 or 6 members is generally substituted with a $C_{1-2}$ hydrocarbon. Also, examples ofthe heterocycle can include a heterocycle group substituted with 1-4 substituents such as halogen (fluorine, chlorine, bromine, iodine and the like), a $C_{1-4}$ alkyl (methyl, ethyl, propyl, isopropyl, and the like) and halogenophenoxy (*o*-, *m*-, or *p*-chlorophenoxy, *o*-, *m*-, or *p*-bromophenoxy and the like).

The alkyl group, alkenyl group, nonaromatic heterocycle and aromatic heterocycle in said $R^7$ and $R^8$ can have

at least one substituent, e.g., 1-5 substituents, at a feasible position. Examples of the substituent include hydroxy, mercapto, cyano, halogen (fluorine, chlorine, bromine, iodine), a $C_{1-4}$ alkoxy (e.g., methoxy, ethoxy, propoxy, isopropoxy, butoxy, isobutoxy, sec-butoxy, *tert*-butoxy, 2-methoxyethoxy and the like), a $C_{1-4}$ alkylthio (e.g., methylthio, ethylthio, *n*-propylthio, isopropylthio, *n*-butylthio, *tert*-butylthio and the like), a $C_{1-4}$ alkylsulfinyl (e.g., methylsulfinyl, ethylsulfinyl and the like), a $C_{1-4}$ alkylsulfonyl (e.g., methylsulfonyl, ethylsulfonyl and the like) and a $C_{1-10}$ haloalkyl (e.g., difluoromethyl, trifluoromethyl, trifluoroethyl, trichloroethyl and the like).

In addition, examples of the substituent can include an unsubstituted or substituted amino, i.e., a $C_{1-6}$ alkanoylamino (e.g., acetylamino, propionylamino and the like), a $C_{1-10}$ alkylamino (e.g., methylamino, ethylamino, *n*-propylamino, isopropylamino, *n*-butylamino, isobutylamino, sec-butylamino, *tert*-butylamino, pentylamino, hexylamino, heptylamino, octylamino, nonylamino, decylamino and the like, in which the alkyl group may be substituted with hydroxy group), a di-$C_{1-4}$ alkylamino (e.g., dimethylamino, diethylamino, *N*-methyl-*N*-ethylamino, *N*-methyl-*N*-propylamino and the like).

Compound (I) can be manufactured by using known reactions. Although the representative synthetic examples will be shown in below, the present invention is not restricted thereto. In the following manufacturing methods, $R^1$ and $R^2$ can be replaced with $R^7$ and $R^8$ to be read. Also, $R^1$, $R^2$, $R^7$ and $R^8$ are as shown in definitions mentioned above, unless otherwise indicated. In order to obtain the compound as an optically active substance, an optically active material, reagent, catalyst or the like may be used. Also, at a suitable step, a separating operation such as chromatography or fractional crystallization may be performed. Further, when there are a conformational isomer and a geometrical isomer, a starting material and a reaction condition can be selected suitably and then separating operation can be performed to obtain a pure conformational isomer or a geometrical isomer. Furthermore, when there is a functional group in the molecule and said functional group becomes or in danger of a disturbance of reaction, it is preferable that a suitable protecting group is used to put the reaction forward effectively. Use of protecting group can be performed, for example, according to Protective Groups in Organic Synthesis by Theodora W.Greene, Peter G.M.Wuts et al.. Also, unless there is any problem, a reaction condition and order of process can be changed, thereby selecting more suitable method.

Scheme 1

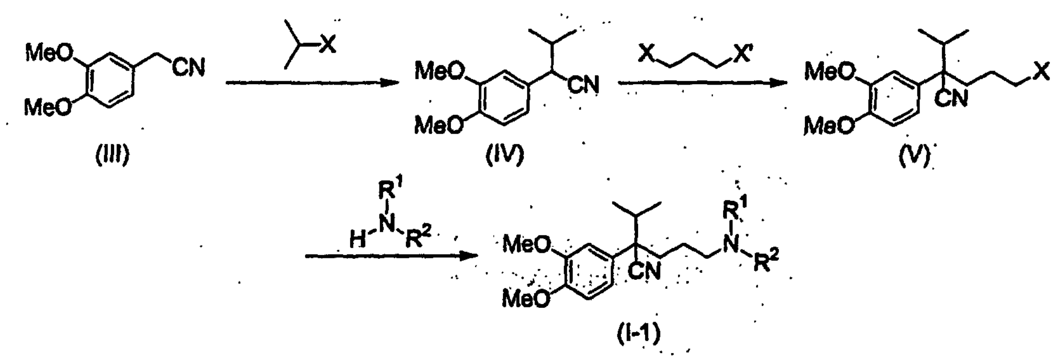

Compound (I-1) can be synthesized, for example, as shown in Scheme 1, wherein dimethoxyphenylacetonitrile (III) is reacted with 2-substituted propane to give Compound (IV), this Compound (IV) is reacted with 1,3-disubstituted propane to give Compound (V), and then Compound (V) is reacted with an amine to give Compound (I-1). Each of X and X' represents a leaving group, which may be identical or different from each other. Examples of the leaving group include a halogen atom such as fluorine, chlorine, bromine, iodine or the like, mesyl group and tosyl group. In the following, the definitions of X and X' is the same unless otherwise specified.

As a 2-substituted propane used in the reaction with dimethoxyphenylacetonitrile (III), for example, 2-chloropropane, 2-bromopropane or 2-iodopropane can be used. The reaction can be usually performed in the presence of a base such as metallic sodium, sodium hydride or sodium amide with or without a solvent. As a solvent, an aromatic carbon such as benzene, toluene or xylene; an ether such as tetrahydrofuran or 1,4-dioxane; an amide such as *N,N*-dimethylformamide, *N,N* dimethylacetamide and hexamethylphosphoramide; acetonitrile, dimethyl sulfoxide and the like can be used. While the reaction temperature and reaction time may be changed according to the starting materials and the reagents used, the reaction is usually effected at a temperature within the range of -15° C to the reflux temperature of the solvent. Specifically, for example, Compound (III) and isopropyl bromide are reacted in tetrahydrofuran

in the presence of sodium amide at a temperature within the range of room temperature to the reflux temperature, thereby attaining the aimed object.

As a 1,3-disubstituted propane used in the reaction with Compound (IV), for example, 1-chloro-3-iodopropane, 1-bromo-3-iodopropane, 1-bromo-3-chloropropane, 1,3-dichloropropane, 1,3-dibromopropane or 1,3-diiodopropane can be used. The reaction can be usually effected in the presence of a base such as metallic sodium, sodium hydride or sodium amide with or without a solvent. As a solvent, an aromatic carbon such as benzene, toluene or xylene; an ether such as tetrahydrofuran or 1,4-dioxane; an amide such as *N,N*-dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of -15°C to the reflux temperature of the solvent. Specifically, for example, Compound (IV) and 1-chloro-3-iodopropane are reacted in toluene in the presence of sodium amide at a temperature within the range of room temperature to the reflux temperature, thereby attaining the aimed object.

The reaction of Compound (V) with the amine $NR^1R^2$ can be usually effected in the presence of a base. As a base, for example, an inorganic base such as sodium hydroxide, potassium hydroxide, sodium carbonate, potassium carbonate, sodium hydrogen carbonate or potassium hydrogen carbonate, or an organic base such as butyl lithium can be used. As a solvent, an aromatic hydrocarbon such as benzene, toluene or xylene; an ether such as tetrahydrofuran or 1,4-dioxane; an alcohol such as methanol or ethanol; an amide such as *N,N*-dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of 0° C to the reflux temperature of the solvent. Specifically, for example, Compound (V) and the amine are reacted in *N,N*-dimethylacetamide in the presence of potassium carbonate at a temperature within the range of room temperature to the reflux temperature, thereby attaining the aimed object.

Also, Compound (I-1) can be synthesized, as shown in Scheme 2, by a reaction of Compound (VI) obtained from amine $NR^1R^2$ and 1,3-disubstituted propane with Compound (IV).

Scheme 2

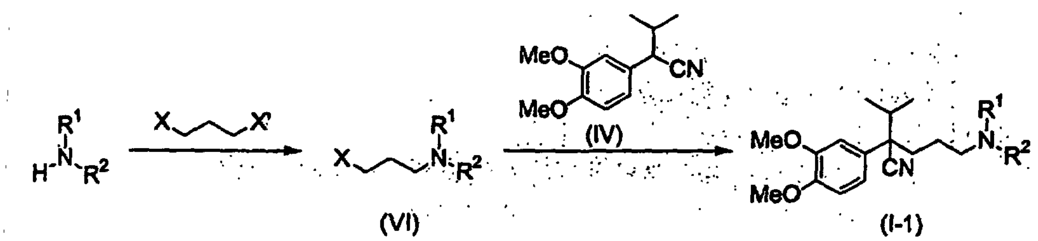

The reaction of the amine $NR^1R^2$ with 1,3-disubstituted propane can be usually effected in the presence of an inorganic base such as sodium hydroxide, potassium hydroxide, sodium carbonate, potassium carbonate, sodium hydrogen carbonate or potassium hydrogen carbonate, or an organic base such as butyl lithium with or without a solvent. As a solvent, an aromatic hydrocarbon such as benzene, toluene or xylene; an ether such as tetrahydrofuran or 1,4-dioxane; an alcohol such as methanol or ethanol; an amide such as *N,N*-dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of 0° C to the reflux temperature of the solvent. Specifically, for example, the amine and 1-chloro-3-iodopropane are reacted in *N,N*-dimethylacetamide in the presence of potassium carbonate at a temperature within the range of room temperature to the reflux temperature, thereby attaining the aimed object.

The reaction of Compound (VI) with Compound (IV) may be effected according to said reaction of Compound (IV) with a 1,3-disubstituted propane. Namely, it can be effected in the presence of a base such as metallic sodium, sodium hydride or sodium amide with or without a solvent. As a solvent, an aromatic hydrocarbon such as benzene, toluene or xylene; an ether such as tetrahydrofuran or 1,4-dioxane; an amide such as *N,N*-dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of -15°C to the reflux temperature of the solvent.

Specifically, for example, Compound (IV) and Compound (VI) are reacted in toluene in the presence of sodium amide at a temperature within the range of room temperature to the reflux temperature, thereby attaining the aimed object.

The synthesis of Compound (I-1) from Compound (V) can be effected by a method shown in Scheme 3.

Scheme3

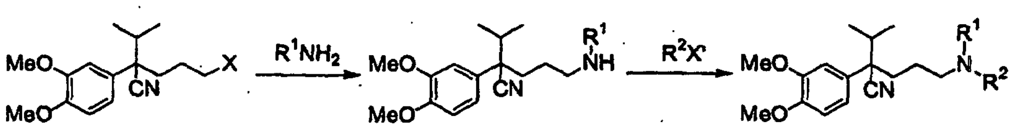


(V) (VII) (I-1)

The reaction of Compound (V) with monoalkylamine can be effected according to the said reaction of Compound (V) with the amine. Namely, it can be effected in the presence of an inorganic base such as sodium hydroxide, potassium hydroxide, sodium carbonate, potassium carbonate, sodium hydrogen carbonate or potassium hydrogen carbonate, or an organic base such as butyl lithium in a solvent. As a solvent, an aromatic hydrocarbon such as benzene, toluene or xylene; an ether such as tetrahydrofuran or 1,4-dioxane; an alcohol such as methanol or ethanol; an amide such as *N,N*-dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of 0°C to the reflux temperature of the solvent. Specifically, for example, Compound (V) and monoalkylamine are reacted in *N,N*-dimethyl acetamide in the presence of potassium carbonate at a temperature within the range of room temperature to the reflux temperature, thereby attaining the aimed object.

The reaction of Compound (VII) with the substituted alkyl is usually effected in the presence of a base with or without a solvent. The base used in this reaction can be an inorganic base such as sodium hydroxide, potassium hydroxide, sodium carbonate, potassium carbonate, sodium hydrogen carbonate or potassium hydrogen carbonate, or an organic base such as butyl lithium. As a solvent, an aromatic hydrocarbon such as benzene, toluene or xylene; an ether such as tetrahydrofuran or 1,4-dioxane; an alcohol such as methanol or ethanol; an amide such as *N,N*-dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of 0°C to the reflux temperature of the solvent. Specifically, for example, Compound (VII) and the alkyl bromide are reacted in *N,N*-dimethylacetamide in the presence of potassium carbonate at a temperature within the range of room temperature to the reflux temperature, thereby attaining the aimed object.

Also, the synthesis of Compound (I-1) from Compound (IV) can be effected by a method shown in Scheme 4.

Scheme 4

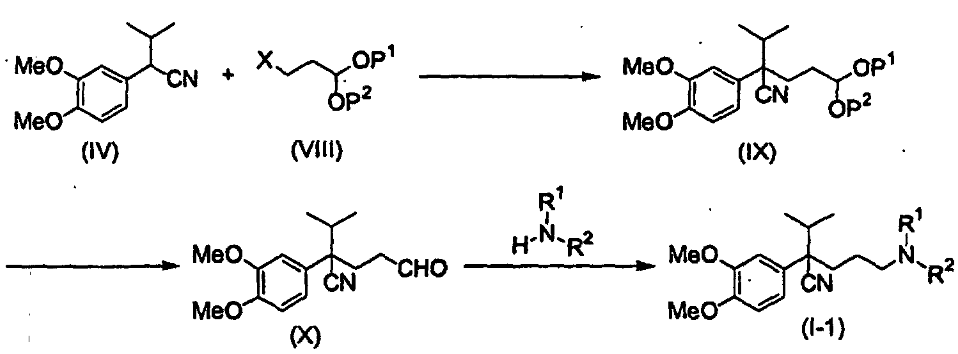

(IV) (VIII) (IX)

(X) (I-1)

The reaction of Compound (IV) with Compound (VIII) is usually effected in the presence of a base. Each of $P^1$ and $P^2$ represents a carbonyl-protecting group, which may be identical or different from each other. Examples of the protecting group include a lower alkyl group. Also, $P^1$ and $P^2$ may together form a $C_{2-6}$ alkylene group. As a base, metallic sodium, sodium hydride, sodium amide or the like can be used. The reaction is effected with or without a solvent, which can be an aromatic hydrocarbon such as benzene, toluene or xylene; an ether such as tetrahydrofuran or 1,4-dioxane; an amide such as *N,N*-dimethylformamide, *N,N*-dimethylacetamide or hexamethylphosphoramide; acetonitrile; dimethyl sulfoxide or the like. While the reaction temperature and reaction time may be changed according to the starting materials and the reagents used, the reaction is usually effected at a temperature within the range of -15°C to the reflux temperature of the solvent. Specifically, for example, Compound (IV) and Compound (VIII) are reacted in toluene in the presence of sodium amide at a temperature within the range of room temperature to the reflux temperature, thereby attaining the aimed object.

The synthesis of Compound (X) by deprotecting Compound (IX) is usually effected in the presence of acid catalyst. As an acid catalyst, an inorganic acid such as hydrochloric acid, sulfuric acid, nitric acid, aluminum chloride, titanium tetrachloride, iron chloride or trifluoroborane, or an organic acid such as oxalic acid or titanium tetraisopropoxide can be used. The reaction is usually effected with or without a solvent. As a solvent, an aromatic hydrocarbon such as benzene, toluene or xylene; an ether such as tetrahydrofuran or 1,4-dioxane; an amide such as *N,N*-dimethylformamide, *N,N*-dimethylacetamide or hexamethylphosphoramide; acetone; acetonitrile; dimethyl sulfoxide and the like can be used. While the reaction temperature and reaction time may be changed according to the starting materials and the reagents used, the reaction is usually effected at a temperature within the range of 0°C to the reflux temperature of the solvent. Specifically, for example, Compound (IX) and oxalic acid are mixed in acetone and reacted at a temperature within the range of room temperature to the reflux temperature, thereby attaining the aimed object.

The synthesis of Compound (I-1) by reductive amination of Compound (X) with the amine is usually effected in the presence of a reducing agent such as sodium borohydride, sodium cyanoborohydride or sodium trimethoxyborohydride with a solvent. As a solvent, an alcohol such as methanol or ethanol; an ether such as tetrahydrofuran or 1,4-dioxane; and amide such as *N,N* dimethylformamide, *N,N*-dimethylacetamide or hexamethylphosphoramide; acetonitrile; dimethyl sulfoxide and the like can be used. While the reaction temperature and reaction time may be changed according to the starting materials and the reagents used, the reaction is usually effected at a temperature within the range of 0°C to the reflux temperature of the solvent. Specifically, for example, Compound (X) and the amine are mixed in methanol and reacted, with sodium cyanoborohydride added thereto little by little, at a temperature within the range of room temperature to the reflux temperature, thereby attaining the aimed object.

In any reaction mentioned above, a catalyst, a halogen-exchanging agent, a pH adjusting agent, a cosolvent or the like can be added, if necessary. The starting materials used in the foregoing Reaction Formulae are commercially available or can be synthesized from a suitable starting material by using known methods.

In the present invention, Compound (I) can be changed to an acid-added salt if necessary. Examples of the acid-added salt include salt with an inorganic acid such as hydrochloric acid, hydrobromic acid, sulfuric acid or phosphoric acid and salts with an organic acid such as acetic acid, propionic acid, citric acid, lactic acid, oxalic acid, maleic acid, fumaric acid, succinic acid, tartaric acid or methanesulfonic acid. These salts can be easily manufactured by common methods.

Compound (I), which mechanism of action has not been made clear, have an excellent hair follicle cell growth-simulative effect. Therefore, it is useful for a hair growth promoting composition (which is a general idea including hair growth composition, hair regrowth promoting composition and the like), which purpose is to promote hair growth and regrowth and to prevent hair loss in human. By applying it on scalp, care, improvement or prevention of hair loss can be expected.

The hair growth promoting composition comprising Compound (I) of the present invention can apply to pathological alopecia such as alopecia areata, alopecia pityrodes or alopecia seborrheica in addition to thin hair or hair loss what is called male pattern baldness or androgenic alopecia. The dosage of the compound (I) must be determined suitably according to sex, age and degree of symptom in hair loss or thin hair. Usually 0.01-20 $mg/cm^2$ is applied on scalp per day for an adult in a single dose or several doses.

The hair promoting composition of the present invention can be used as a drug, quasi-drug or cosmetic external preparation, which purpose is to promote hair growth and regrowth and to prevent hair loss. The pharmaceutical form can be selected voluntarily as long as the effects of the present invention can be exhibited. Examples of the pharmaceutical form include a tonic, a lotion, a milky lotion, a cream, an ointment, a gel, an aerosol, a spray and a mousse. The product form can be also selected freely. For example, it may be a hair regrowth promoting composition, a hair growth composition, a scalp treatment, a hair tonic, a shampoo, a rinse, a hair pack, a hair lotion, a conditioner, and a scalp treatment.

Compound (I), which is not restricted as long as the present effect is spoiled, is usually 0.001 - 20 % by weight, preferably 0.01 - 5 % by weight in the hair growth promoting composition or external preparation for skin of the present invention. When the compounding amount is too less, it may be lead to an insufficient effect of the present invention.

On the other hand, when the compounding amount is too much, it may be lead to a pharmaceutically unfavorable composition. In the present invention, two or more of Compound (I) may be compounded in combination.

In addition to Compound (I), if necessary, ingredients normally used in the field of drug, quasi-drug and cosmetic can be compounded to the hair growth promoting composition and external preparation for skin of the present invention as long as the present effect is not spoiled. Examples of drugs having a blood flow promoting action include swertia herb extract, vitamin E and derivatives thereof, nicotinates such as benzyl nicotinate. Examples of drugs that promote blood circulation by topical stimulation include capsicum tincture, cantharides tincture, camphor and vanillic acid nonylamide. Examples of drugs having hair follicle activating action include hinokitiol, placental extract, photosensitizing dye, pantothenic acid and derivatives thereof. Examples of drugs having antiandrogen action include a hormone such as estradiol or estrone. Examples of drugs having antiseborrheic action include sulfur, thioxolone and vitamin $B_5$.

In addition, salicylic acid, resorcine and the like which has corneocycle desquamating and antibacterial action can be compounded therein so as to prevent the generation of dandruff. Also, glycyrrhizic acid and derivatives thereof, menthol, and the like can be compounded therein so as to prevent inflammation of scalp. Further, an amino acid such as serine, methionine or arginine, a vitamin such as biotin, extracts of crude drugs and the like can be compounded therein in order to supplement nutrition for hair follicle and activate enzyme activity.

Also, extracts from plants such as althea, coix, peppermint, leaf base, capsicum, aloe, lycium, mugwort, oryza, seashore vitex, rosmarinus officinalis, drynaria, cytisus scoparius, gentiana, salviae miltiorrhizeae radix, sponge gourd, platycodon, pinus, sophora root, Japanese angelica root, safflower, Japanese barberry, areca, eucalyptus, prunella spike, akebia stem, achyranthes root, bupleurum root, tea, licorice, hop, Chrysanthemum, senega, sesame, cnidium rhizome, cashew, pueraria root, rosae rugosae flos, saffron, rosemary, rehmannia root, or mallow can be compounded.

Also, a vasodilator such as alkoxycarbonylpyridine *N*-oxide, carpronium chloride or acetylcholine derivative; a cutaneous hyperfunctioning agent such as cephalanthin; an antibacterial agent such as hexachlorophene, benzalkonium chloride, cetylpyridinium chloride, undecylenic acid, trichlorocarbanilide or bithionol; zinc and its derivatives; lactic acid and its alkyl ester; an organic acid such as citric acid; a protease inhibitor such as tranexamic acid; and the like can be compounded. Other drugs may be compounded thereto.

Further, an alcohol such as ethanol or isopropanol; a polyvalent alcohol such as glycerin, propylene glycol or polyethylene glycol; an oily ingredient such as higher fatty acids, higher alcohols, hydrocarbons, natural oils and fats, ester oils or silicone oils; surfactants; perfumes; chelating agents; humectants such as 1,3-butyleneglycol, hyaluronic acid and its derivatives, maltitol, soluble collagen or sodium lactate; thickening agents such as quince mucilage, carboxyvinyl polymer or xanthan gum; antioxidants; ultraviolet absorbers: coloring agents; water; stabilizers; powders; water-soluble macromolecular compounds; coating materials; and the like can be compounded.

## EXAMPLES

In the following, the present invention will be further explained by specific examples. However, the present invention should not be restricted thereto. The compounding amount and concentration were shown by weight % unless otherwise specified.

### Experiment 1

Growth-stimulative activity test on immortalized human outer root sheath cell

(1) Immortalized human outer root sheath cell

Keratinocyte-SFM medium (Gibco BRL), to which a supplement attached with the medium was added, was used for culture on a dish which surface was coated with collagen. The cell was cultured until it became semiconfluent and then peeled off with 0.05% trypsin/PBS(-) and subcultured. 30-$40^{th}$ stage culture cells were used for study on growth.

(2) Sample Preparation

Each tested compound described below is dissolved with distilled water at the concentration of 10mM and sterilized through Millipore filter. This aqueous solution was diluted with KBM at a predetermined concentration and added to the cell as a sample.

Tested Compound

Example 21:

2-(3' ,4'-dimethoxyphenyl)-2-isopropyl-5-[methyl(3-methylbutyl)amino]-pentanenitrile hydrochloride

Example 22:

(*R*)-2-(3' ,4' -dimethoxyphenyl)-2-isopropyl-5-(methylamino)pentanenitrile hydrochloride

Example 23:

(*R*)-2-(3' ,4' -dimethoxyphenyl)-2-isopropyl-5-[methyl(3-methylbutyl)amino]-pentanenitrile hydrochloride

(3) Evaluation of growth-stimulative activity on immortalized human outer root sheath cell

The immortalized human outer root sheath cell was inoculated into a microplate (Iwaki, 24 wells) (20000cells/well) with a growth medium KGM (Sanko Junyaku Co., Ltd., CC-3111) and incubated at 37°C for 1 day. After each well was washed once with basal medium KBM (Sanko Junyaku Co., Ltd., CC-3101), the medium was exchanged with KBM containing each sample, and then incubated further for 2 days. [$^3$H]-Thymidine (Amersham Pharmacia Biotech K.K., TRK758, the final concentration: 20 μCi/ml) was added thereto and incubated at 37°C for 3 hours. The cell was peeled off with 0.25% trypsin/PBS(-) and collected on a glass filter (Watmann, GLC). After [$^3$H]-thymidine that is not taken into the cell was removed by washing with distilled water, the filter was air-dried and then the radioactivity was measured by a liquid scintillation counter.

(4) Result

In a experiment adding each sample at a concentration within a range of $10^{-9}$ - $10^{-13}$M, using the uptake quantity of radioactivity in the sample-added group when that in the negative control medium (KBM+d$H_2$O) was regarded as 100%, the immortalized human outer root sheath cell growth activity was evaluated. The results were shown in Table 1.

Table 1

| Tested compound | Concentration | [$^3$H]-thymidine uptake quantity |
|---|---|---|
| Negative control | - | 100% |
| Example 21 | $10^{-9}$ M | 151% |
| " | $10^{-10}$M | 143% |
| " | $10^{-11}$M | 160% |
| " | $10^{-12}$M | 114% |
| " | $10^{-13}$M | 118% |
| Example 22 | $10^{-9}$ M | 105% |
| " | $10^{-10}$M | 98% |
| " | $10^{-11}$M | 111% |
| " | $10^{-12}$M | 135% |
| " | $10^{-13}$M | 153% |
| " | $10^{-14}$M | 160% |
| Example 23 | $10^{-9}$ M | 115% |
| " | $10^{-10}$M | 106% |
| " | $10^{-11}$M | 107% |
| " | $10^{-12}$M | 117% |
| " | $10^{-13}$M | 114% |
| " | $10^{-14}$M | 125% |

As evident from Table 1 above, it can be understood that a phenylacetonitrile derivative in accordance with

the present invention shows a growth-stimulative activity on immortalized human outer root sheath cell and is useful as a hair growth promoter.

Experiment 2

Growth-stimulative activity test on artificial human bulbus pili cell

(1) Preparation of first stage incubated outer root sheath cell

A bulbus pili site of a hair follicle isolated from a human scalp obtained by a plastic surgery was excised and then enzymatically treated in Keratinocyte-SFM medium(GibcoBRL : 11965-011) supplemented with 1000unit/ml dispase + 0.2% collagenase at 37 °C for 30 minutes. After unnecessary hair follicle mesenchymal tissue such as connective tissue root sheath was removed therefrom using the tip of a syringe needle (27G), the residue was placed in the incubating space of an incubator coated with collagen and explant culture was performed in Keratinocyte-SFM medium supplemented with an antibacterial agent. After incubation for about 1 week, the medium was exchanged upon ensuring the growth of the cells. Cells grown to be subconfluent were treated with PBS(-) + 0.05% trypsin at 37 °C for 3 minutes and the reaction was stopped with an equivalent amount or more of PBS(-) + 0.1% trypsin inhibitor (Sigma). The cells were collected by centrifugal separation (1200rpm x 5min) and suspended again in PBS(-). The cells were collected by centrifugal separation (1200rpm x 5min), thereby washing the cells. Washed cells were stored while being frozen in a liquid nitrogen using Cellbanker II (DIA-IATRON : ZCB-201(100)), thereby obtaining the first stage incubated outer root sheath cells.

(2) Preparation of first stage incubated dermal papilla cell

From a bulbus pili site obtained by excising a hair follicle isolated from a human scalp obtained by a plastic surgery, using the tip of a syringe needle (27G), a dermal papilla site was isolated under a stereoscopic microscope and placed in the incubating space of an incubator coated with collagen. Then, explant culture was performed in 10%FBS-added DMEM medium (GibcoBRL : 11965-092) supplemented with an antibacterial agent. After incubation for about 2 week, the medium was exchanged upon ensuring the growth of the cells. Cells grown to be subconfluent were treated with PBS(-) + 0.05% trypsin at 37°C for 3 minutes and the reaction was stopped with an equivalent amount or more of PB S(-) + 0.1%trypsin inhibitor (Sigma). The cells were collected by centrifugal separation (1200rpm x 5min) and suspended again in PBS(-). The cells were collected by centrifugal separation (1200rpm X 5min), thereby washing the cells. Washed cells were stored while being frozen in a liquid nitrogen using Cellbanker II (DIA-IATRON : ZCB-201 (100)), thereby obtaining the first stage incubated dermal papilla cells.

(3) Manufacture of the artificial bulbus pili site

The first stage incubated dermal papilla cell obtained above was subcultured three times (37°C, 5% $CO_2$) in a 10% FBS-supplemented DMEM medium (GibcoBRL : 11965-092) in a 75 $cm^2$ incubation flask coated with Type I collagen, and then stored while being frozen in a liquid nitrogen using Cellbanker II (DIA-IATRON : ZCB-201(100)) (DPc).

Also, the first stage incubated outer root sheath cell obtained above was subcultured three times (37°C, 5% $CO_2$) in a Keratinocyte-SFM medium (GibcoBRL: 11965-011) in a 75 $cm^2$ incubation flask coated with Type I collagen, and then stored while being frozen in a liquid nitrogen using Cellbanker II (DIA-IATRON : ZCB-201(100)) (ORSc).

The DPc and ORSc stored as frozen were thawed, washed with an ice-cooled Keratinocyte-SFM medium, dispersed in Keratinocyte-SFM(+) medium (which is Keratinocyte-SFM medium supplemented with 5ng/mL epidermal growth factor and 5µl/mL bovine pituitary glanusi extract), and subjected to an accurate cell density measurement using a hemocytometer. Then, each cell density was adjusted at 5 x $10^4$ cells/ml, and the cell dispersions in equal volumes were combined under cooling with ice. 80 µl aliquots of the combined cell suspending Keratinocyte-SFM(+) medium were inoculated into individual wells of Sumilon celltight spheroid 96U plate (Sumitomo Bakelit co.,), and incubated for 2 days (37°C, 5% $CO_2$). A cell in a condition that an ORSc was attached around a cell cluster of DPc was sorted out, to manufacture the artificial bulbus pili site by one-step process.

(4) Addition of test material and measurement of activity

Each 80 µl of a test material-supplemented William's E(+) medium (which is William's E medium supplemented with 10 µg/ml transferrin, 10 ng/ml hydrocortisone, 10 µg/ml insulin and 10 ng/ml sodium selenite) was added to each well and incubated at 37°C under 5% $CO_2$ further for 2 days.

The test material-supplemented William's E(+) medium was prepared by dissolving the test material in ethanol at a concentration of $10^{-9}$ M or $10^{-11}$ M and adding to William's E(+) medium at 0.2% each. Accordingly, the final concentration of the test material in each well was $10^{-12}$ M or $10^{-14}$ M, and each final concentration of ethanol was 0.1 %.
As a negative control, 80 μl of William's E(+) medium containing 0.2% ethanol was added (the final concentration of ethanol was 0.1%).
After incubation, Alamer Blue (Biosource: DAL 1100), Keratinocyte-SFM medium and William's E(+) medium were mixed in 2:1:1 ratio (volume ratio), warmed at 37°C and then 40 μl aliquots were added to individual wells and allowed to react at 37°C for 6 hours. After completion of the reaction, 100 μl aliquots were taken from individual wells, and transferred to each well of a white 96-well plate for fluorescence measurement (Opaque Plate) (Coster:3912).
A fluorometer (Labsystems Fluoroskan II) was used at the excitation wavelength of 544 nm and the fluorescent wavelength of 590 nm to measure the fluorescence intensity of each well, from which the fluorescent intensity of a cell-free reaction mixture (i.e., the mixture itself) was subtracted to obtain a respiration level under each condition. A relative respiration level (%) was calculated according to the following equation.

Relative respiration level (%)

={Respiration level (test material)} / {Respiration level (negative control)} x 100

Using the relative respiration level as an index, the cell growth-stimulative effect on an artificial bulbus pili site was judged. Judgment standard is as follows:

- : No effect (the relative respiration level is less than 102%).
± : Weak effect is recognized (102% or more, and less then 105%).
+ : Effect is recognized (105% or more, and less then 110%).
++ : Strong effect is recognized (110% or more, and less than 115%).
+++ : Very strong effect is recognized (115% or more).

(5) Result

Table 2

| Example No. | Judgment |
|---|---|
| 4 | +++ |
| 5 | + |
| 6 | + |
| 7 | + |
| 8 | ++ |
| 9 | + |
| 12 | + |
| 15 | + |
| 16 | + |
| 17 | ++ |
| 18 | +++ |
| 24 | ± |
| 25 | ++ |
| 26 | + |
| 27 | + |
| 28 | ++ |

As evident from Table 2 above, it was suggested that a phenylacetonitrile derivative of the present invention showed a cell growth-stimulative activity in the test using an artificial bulbus pili site and was useful as an hair growth promoter. Especially, Examples 8, 17,25 and 28 showed strong effects, and Examples 4 and 18 showed very strong effects.

Example 1

Synthesis of 2-(3',4'-dimethoxyphenyl)-2-isopropyl-5-methylaminopentanenitrile

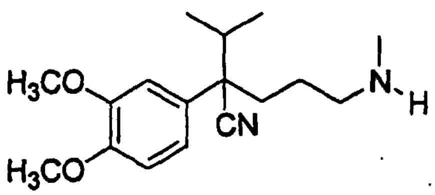

(1) Synthesis of 2-(3' ,4' -dimethoxyphenyl)-3-methylbutyronitrile

17.70g(100mmol) of 3,4-dimethoxyacetonitrile was dissolved in 100ml of anhydrous tetrahydrofuran, and 4.68g(120mmol) of sodium amide was added thereto in fractional amounts while being stirred and cooled with ice. After being stirred for 30 minutes, 14.76g(120mmol) of 2-bromopropane was dropwise added to the mixture and then stirred with heating at 60°C for 1 hour. After disappearance of the starting material was checked, a small amount of methanol was added to the mixture to decompose excess sodium amide, and the solvent was evaporated out under a vacuum. The residue was extracted with ethyl acetate and then washed with water until it became neutral. The organic layer was filtrated through IPS filter paper, and then the solvent was evaporated out. The residue was purified by silica gel column chromatography, to give 17.8g(81mmol, 81%) of 2-(3',4'-dimethoxyphenyl)-3-methylbtyronitrile.
$^1$H-NMR (d, $CDCl_3$):1.05 (6H, d, *J*=6.6Hz), 2.00-2.15 (1H, m), 3.59 (1H, d, *J*=6.6Hz), 3.89 (3H, s), 3.90 (3H, s), 6.76-6.86 (3H, m).

(2) Synthesis of 5-chloro-2-(3',4' -dimethoxyphenyl)-2-isopropylpentanenitrile

10.16g(50mmol) of 2-(3' ,4' -dimethoxyphenyl)-3-methylbutyronitrile was dissolved in 80ml of anhydrous toluene, and 3.90g(100mmol) of sodium amide was added in fractional amounts while being stirred. After being heated and refluxed at 120 ° C for 2 hours, the mixture was cooled down to 80°C and, with 15.33g(75mmol) of 1-chloro-3-iodopropane added thereto, further stirred at 80 ° C for 1 hour. After disappearance of the starting material was checked, a small amount of methanol was added to the mixture to decompose excess sodium amide, and the solvent was evaporated out under a vacuum. The residue was extracted with ethyl acetate and then washed with water until it became neutral. The organic layer was filtrated through 1PS filter paper, and then the solvent was evaporated out. The residue was purified by silica gel column chromatography, to give 12.03g(41mmol, 81%) of 5-chloro-2-(3',4' -dimethoxyphenyl)-2-isopropylpentanenitrile.
$^1$H-NMR (d, $CDCl_3$):0.82 (3H, d, *J*=6.7Hz), 1.21 (3H, d, *J*=6.7Hz), 1.38-1.54 (1H, m), 1.80-1.98 (1H, m), 2.00-2.30 (3H, m), 3.45-3.54 (2H, m), 3.89 (3H, s), 3.90 (3H, s), 6.82-6.96 (3H, m).

(3) Synthesis of 2-(3',4' -dimethoxyphenyl)-2-isopropyl-5-methylaminopentanenitrile

10.40g(35mmol) of 5-chloro-2-(3' ,4' -dimethoxyphenyl)-2-isopropylpentanenitrile was dissolved in 50ml of anhydrous ethanol, and 14.50g(105mmol) of potassium carbonate, 18.00g(175mmol) of 30% methylamine methanol solution and 0.50g of sodium iodide were added thereto. Then, the mixture was sealed in a tube and stirred at 100°C for 72 hours. After the solvent was evaporated out under a vacuum, the residue was extracted with ethyl acetate and washed with water three times until it became neutral. The organic layer was acidified with 50ml of 2N hydrochloric acid, and the water layer was collected. After further three times extraction with 2N hydrochloric acid, the water layers were combined and basified with 48% sodium hydroxide aqueous solution while being cooled with ice, and then extracted three times with 50 of ethyl acetate. The organic layer was filtrated through 1PS filter paper, and the solvent was evaporated out, to give 7.36g(25mmol, 72%) of 2-(3' ,4' -dimethoxyphenyl)-2-isopropyl-5-methylaminopentanenitrile.
$^1$H-NMR (d, $CDCl_3$):0.80 (3H, d, *J*=6.5Hz), 1.20 (3H, d, *J*=6.5Hz), 1.14-1.23 (1H, m), 1.54-1.61 (1H, m), 1.86-1.95 (1H, m), 2.08 (1H, sept, *J*=6.5Hz), 2.14-2.22 (1H, m), 2.27 (1H, br-s), 2.37 (3H, s), 2.51-2.61 (2H, m), 3.88 (3H, s), 3.90 (3H, s), 6.83-6.6.87 (2H, m), 6.91-6.95 (1H, m).

Example 2

Synthesis of 2-(3',4' -dimethoxyphenyl)-2-isopropyl-5-[*N*-methyl-*N*-(3-methylbutyl)amino]-pentanenitrile

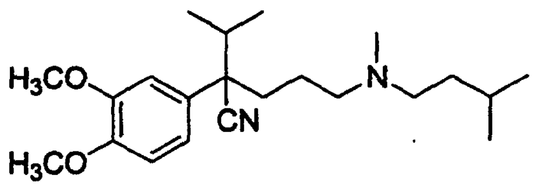

2.03g(7.0mmol) of 2-(3' ,4' -dimethoxyphenyl)-2-isopropyl-5-methylamino pentanenitrilewas dissolved in 7ml of dimethylformamide, and 1.45g(10.5mmol) of potassium carbonate was added thereto. After being stirred at room temperature for 10 minutes, the mixture, with 1.59g(10.5mmol) of isopentyl bromide added thereto, was stirred at room temperature for 16 hours. The solvent was evaporated out under a vacuum, and the residue was extracted with dichloromethane and washed with water until it became neutral. The organic layer was filtrated through 1PS filter paper, and the solvent was evaporated out. The residue was purified by silica gel column chromatography, to give 1.84g(5.1mmol, 73%) of 2-(3',4'-dimethoxyphenyl)-2-isopropyl-5-[*N*-methyl-*N*-(3-methylbutyl)amino]- pentanenitrile.
$^1$H-NMR (d, $CDCl_3$):0.80 (3H, d, *J*=6.8Hz), 0.86 (3H, d, *J*=6.8Hz), 0.87 (3H, d, *J*=6.8Hz), 1.10-1.19 (1H, m), 1.19 (3H, d, *J*=6.8Hz), 1.25-1.32 (2H, m), 1.46-1.57 (2H, m), 1.84 (1H, dt, *J*=4.4, 12.2Hz), 2.08 (3H, s), 2.03-2.34 (6H, m), 3.88 (3H, s), 3.89 (3H, s), 6.83-6.87 (2H, m), 6.91-6.95 (1H, m).

Examples 3-10

In Example 2, in the place of isopentyl bromide, an alkyl halide shown in Table 2 below can be used to synthesize each compound of Examples 3-10. In the Table, Ms represents mesyl group.

# Table 2

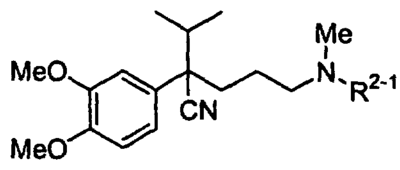

| Example | $R^{2-1}$ | Alkyl halide |
|---|---|---|
| 3 | -Me | MeI |
| 4 | -Et | EtBr |
| 5 | -*n*-Pr | *n*-PrBr |
| 6 | -*i*-Pr | *i*-PrBr |
| 7 | -*n*-Bu | *n*-BuBr |
| 8 | -*i*-Bu | *i*-BuBr |
| 9 | -EtOEt | EtOEtCl |
| 10 | -EtOEtOMe | MeOEtOEtOMs |

The $^1$H-NMR chemical shifts of Examples 3-10 are shown below.

Example 3

$^1$H-NMR (d, $CDCl_3$):0.79 (3H, d, *J*=6.8Hz), 1.09-1.18 (1H, m), 1.20 (3H, d, *J*=6.8Hz), 1.49-1.57 (1H, m) 1.89 (1H, dt, *J*=4.4,12.8Hz), 2.09 (1H, sept, *J*=6.8Hz), 2.12 (6H, s), 2.08-2.21 (2H, m), 2.28 (1H, dt, *J*=12.2, 7.3Hz), 3.89 (3H, s), 3.89 (3H, s), 6.83-6.86 (2H, m), 6.92-6.96 (1H, m).

Example 4

$^1$H-NMR (d, $CDCl_3$):0.79 (3H, d, *J*=6.8Hz), 1.00 (3H, t, *J*=6.8Hz), 1.11-1.20 (1H, m) 1.19 (3H, d, *J*=6.8Hz), 1.50-1.58 (1H, m), 1.86 (1H, dt, *J*=4.4, 12.2Hz), 2.09 (3H, s), 2.03-2.16 (2H, m), 2.21-2.37 (4H, m), 3.89 (3H, s), 3.89 (3H, s), 6.84-6.86 (2H, m), 6.91-6.95 (1H, m).

Example 5

$^1$H-NMR (d, $CDCl_3$):0.80 (3H, d, *J*=6.8Hz), 0.85 (3H, t, *J*=7.3Hz), 1.07-1.21 (1H, m) 1.19 (3H, d, *J*=6.8Hz), 1.36-1.57 (3H, m), 1.85 (1H, dt, *J*=4.4, 12.7Hz), 2.09 (3H, s), 2.03-2.34 (6H, m), 3.89 (3H, s), 3.89 (3H, s), 6.83-6.86 (2H, m), 6.91-6.96 (1H, m).

Example 6

$^1$H-NMR (d, $CDCl_3$):0.80 (3H, d, *J*=6.8Hz), 0.93 (3H, d, *J*=6.3Hz), 0.94 (3H, d, *J*=6.3Hz), 1.06-1.18 (1H, m) 1.19 (3H, d, *J*=6.8Hz), 1.45-1.56 (1H, m), 1.86 (1H, dt, *J*=4.4, 12.7Hz), 2.03-2.15 (2H, m), 2.06 (3H, s), 2.31 (3H, t, *J*=7.0Hz), 2.72 (1H, sept, *J*=6.3Hz), 3.88 (3H, s), 3.89 (3H, s), 6.82-6.86 (2H, m), 6.92-6.94 (1H, m).

Example 7

$^1$H-NMR (d, $CDCl_3$):0.80 (3H, d, *J*=6.8Hz), 0.89 (3H, t, *J*=7.3Hz), 1.08-1.22 (1H, m) 1.19 (3H, d, *J*=6.8Hz), 1.23-1.30 (2H, m), 1.33-1.43 (2H, m), 1.45-1.57 (1H, m), 1.85 (1H, dt, *J*=4.4,12.7Hz), 2.03-2.34 (6H, m), 2.09 (3H, s), 3.88 (3H, s), 3.89(3H, s), 6.83-6.86 (2H, m), 6.91-6.94 (1H, m).

Example 8

$^1$H-NMR (d, $CDCl_3$):0.80 (3H, d, *J*=6.8Hz), 0.85 (6H, t, *J*=6.3Hz), 1.06-1.17 (1H, m) 1.19 (3H, d, *J*=6.8Hz), 1.43-1.61 (1H, m), 1.68 (1H, sept, *J*=6.8Hz), 1.86 (1H, dt, *J*=4.4,12.2Hz), 1.95 (2H, d, *J*=7.3Hz), 2.05 (3H, s), 2.05-2.28 (4H, m), 3.89 (3H, s), 3.90 (3H, s), 6.83-6.87 (2H, m), 6.91-6.95 (1H,m).

Example 9

$^1$H-NMR (d, $CDCl_3$):0.79 (3H, d, *J*=6.8Hz), 1.08-1.19 (1H, m), 1.19 (3H, t, *J*=7.0Hz), 1.19 (3H, d, *J*=6.8Hz), 1.47-1.57 (1H, m), 1.87 (1H, dt, *J*=4.4, 12.2Hz), 2.03-2.15 (2H, m), 2.16 (3H, s), 2.27-2.53 (4H, m), 3.44-3.50 (4H, m), 3.89 (3H, s), 3.89 (3H, s), 6.82-6.86 (2H, m), 6.91-6.95 (1H, m).

Example 10

$^1$H-NMR (d, $CDCl_3$):0.79 (3H, d, *J*=6.8Hz), 1.10-1.19 (1H, m) 1.19 (3H, d, *J*=6.8Hz), 1.46-1.57 (1H, m), 1.86 (1H, dt, *J*=4.4,12.7Hz), 2.07 (1H, sept, *J*=6.8Hz), 2.08-2.17 (1H,m), 2.15 (3H, s), 2.27-2.40 (2H, m), 2.43-2.56 (2H, m), 3.37 (3H, s), 3.50-3.59 (6H, m), 3.89 (3H, s), 3.89 (3H, s), 6.83-6.86 (2H, m), 6.91-6.94 (1H, m).

Example 11

Synthesis of 2-(3',4'-dimethoxyphenyl)-2-isopropyl-5-(*N,N*-diethylamino)-pentanenitrile

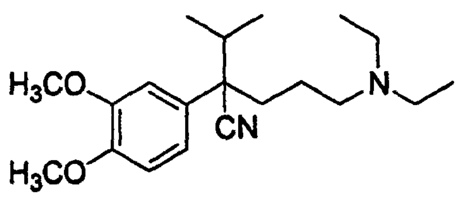

1.00g(3.4mmol) of 5-chloro-2-(3' ,4' -dimethoxyphenyl)-2-isopropylpentanenitrile was dissolved in *N,N*-dimethylformamide, and 1.74g(12.6mmol) of potassium carbonate, 0.92g(12.6mmol) of diethylamine and 0.05g of sodium iodide were added thereto. After being stirred at 100°C for 72 hours, the solvent was evaporated out under a vacuum. The residue was extracted with ethyl acetate and washed three times with water until it became neutral. The organic layer was filtrated through 1 PS filter paper, and the solvent was evaporated put. The residue was purified by silica gel column chromatography, to give 1.08g(3.3mmol, 96%) of 2-(3',4'-dimethoxyphenyl)-2-isopropyl-5-(*N,N*-diethylamino)- pentanenitrile.
$^1$H-NMR (d, $CDCl_3$):0.80 (3H, d, *J*=6.8Hz), 0.95 (6H, t, *J*=7.0Hz), 1.08-1.21 (1H, m), 1.19 (3H, d, *J*=6.8Hz), 1.45-1.57 (1H, m), 1.85 (1H, dt, *J*=4.7, 12.8Hz), 2.03-2.15 (2H, m), 2.32-2.49 (6H, m), 3.89 (3H, s), 3.89 (3H, s), 6.83-6.86 (2H, m), 6.91-6.95 (1H, m).

Examples 12-18

In Example 11, in the place of diethylamine, a dialkylamine shown in Table 3 below can be used to synthesize each compound of Examples 12-15.

## Table 3

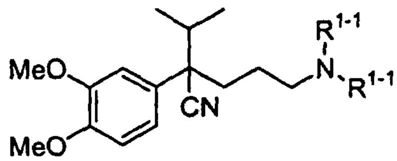

| Example | $R^{1-1}$ | Dialkylamine |
|---|---|---|
| 12 | -*n*-Pr | *n*-$Pr_2NH$ |
| 13 | -*i*-Pr | *i*-$Pr_2NH$ |
| 14 | -*n*-Bu | *n*-$Bu_2NH$ |
| 15 | -*i*-Bu | *i*-$Bu_2NH$ |

Also, in Example 11, in the place of diethylamine, a cyclic amine shown in Table 4 can be used to synthesize each compound of Examples 16-18.

## Table 4

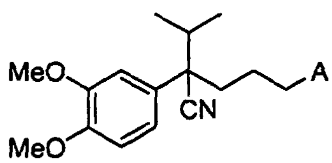

| Example | A | Cyclic amine |
|---|---|---|
| 16 | 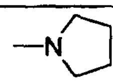 | 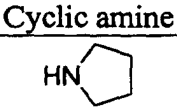 |
| 17 | 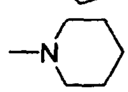 | 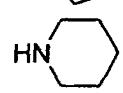 |
| 18 | 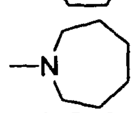 | 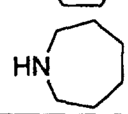 |

The $^1$H-NMR chemical shifts of Examples 12-18 are shown in below.

Example 12

$^1$H-NMR (d, $CDCl_3$):0.80 (3H, d, *J*=6.8Hz), 0.83 (6H, t, *J*=7.3Hz), 1.06-1.17 (1H, m), 1.19 (3H, d,*J*=6.8Hz), 1.31-1.54 (5H, m), 1.83 (1H, dt, *J*=4.4, 12.8Hz), 2.07 (1H, sept, *J*=6.8Hz), 2.08-2.16 (1H, m), 2.24 (2H, t, *J*=7.3Hz), 2.25 (2H, t, *J*=7.3Hz), 2.34 (2H, t, *J*=6.8Hz), 3.89 (3H, s), 3.90 (3H, s), 6.83-6.87 (2H, m), 6.91-6.94 (1H, m).

Example 13

$^1$H-NMR (d, $CDCl_3$):0.80 (3H, d, *J*=6.8Hz), 0.93 (6H, d, *J*=6.3Hz), 0.93 (6H, d, *J*=6.3Hz), 1.01-1.13 (1H, m), 1.18 (3H, d, *J*=6.8Hz), 1.36-1.50 (1H, m), 1.84 (1H, dt, *J*=4.4, 12.7Hz), 2.06 (1H, sept, *J*=6.8Hz), 2.07-2.16 (1H, m), 2.26-2.45 (2H, m), 2.82-2.96 (2H, m), 3.88 (3H, s), 3.89 (3H, s), 6.82-6.86 (2H, m), 6.91-6.94 (1H, m).

Example 14

$^1$H-NMR (d, $CDCl_3$):0.80 (3H, d, *J*=6.8Hz), 0.88 (6H, t, *J*=7.3Hz), 1.06-1.38 (9H, m), 1.19 (3H, d, *J*=6.8Hz), 1.42-1.54 (1H, m), 1.78-1.86 (1H, m), 2.04-2.17 (2H, m), 2.21-2.39 (6H, m), 3.88 (3H, s), 3.90 (3H, s), 6.83-6.87 (2H, m), 6.91-6.94 (1H, m).

Example 15

$^1$H-NMR (d, $CDCl_3$):0.80 (3H, d, *J*=6.8Hz), 0.83 (6H, d, *J*=6.3Hz), 0.85 (6H, d, *J*=6.3Hz), 0.99-1.11 (1H, m), 1.19 (3H, d, *J*=6.8Hz),1.38-1.51 (1H, m), 1.57-1.68 (2H, m), 1.80 (1H, dt, *J*=4.4,12.2Hz), 1.90-2.00 (4H, m), 2.08 (1H, sept, *J*=6.8Hz), 2.18-2.27 (3H, m), 3.89 (3H, s), 3.90 (3H, s), 6.83-6.87 (2H, m), 6.90-6.94 (1H, m).

Example 16

$^1$H-NMR (d, $CDCl_3$):0.79 (3H, d, *J*=6.8Hz), 1.11-1.25 (1H, m), 1.20 (3H, d, *J*=6.8Hz), 1.51-1.64 (1H, m), 1.68-1.80 (4H, m), 1.89 (1H, dt, *J*=4.4, 12.8Hz), 2.08 (1H, sept, *J*=6.8Hz), 2.15 (1H, dt, *J*=4.4,13.1Hz), 2.28-2.51 (6H, m), 3.89 (3H, s), 3.89 (3H, s), 6.82-6.87 (2H, m), 6.91-6.96 (1H, m).

Example 17

$^1$H-NMR (d, $CDCl_3$):0.79 (3H, d, *J*=6.8Hz), 1.10-1.20 (1H, m), 1.19 (3H, d, *J*=6.8Hz), 1.36-1.43 (2H, m), 1.46-1.62 (5H, m), 1.83 (1H, dt, *J*=4.4, 12.8Hz), 2.04-2.14 (2H, m), 2.16-2.32 (6H, m), 3.89 (3H, s), 3.89 (3H, s), 6.82-6.85 (2H, m), 6.90-6.94 (1H, m).

Example 18

$^1$H-NMR (d, $CDCl_3$):0.79 (3H, d, *J*=6.8Hz), 1.09-1.20 (1H, m), 1.19 (3H, d,*J*=6.8Hz), 1.46-1.63 (9H, m), 1.86 (1H, dt, *J*=4.4, 12.3Hz), 2.02-2.16 (2H, m), 2.35-2.58 (6H, m), 3.89 (3H, s), 4.00 (3H, s), 6.82-6.87 (2H, m), 6.91-6.94 (1H, m).

Example 19

Synthesis of (*R*)-2-(3' ,4' -dimethoxyphenyl)-2-isopropyl-5-methylaminopentanenitrile

5.80g(20.0mmol) of 2-(3' ,4' -dimethoxyphenyl)-2-isopropyl-5-methylamino pentanenitrile was dissolved in 50ml of isopropanol, and 7.73g(20.0mmol) of di-*p*-toluoyl-D-tartaric acid was added thereto while being stirred. The mixture was cooled at -20°C for 1 day, and the depositing crystals were collected by filtration. The first and second crystals were combined and suspended again in isopropanol and washed. The organic layers obtained by filtration were combined and the solvent was evaporated out. 50ml of water was added to the residue and then 10ml of 48% sodium hydroxide solution was further added while being cooled with ice. After being stirred for 10 minutes, the reaction mixture was extracted twice with 100ml of ethyl acetate, and then washed with water. The organic layer was filtrated through 1PS filter paper and the solvent was evaporated out. The residue was purified by silica gel column chromatography, and dissolved in 20ml of isopropanol. 2.90g(7.5mmol) of di-*p*-toluoyl-L-tartaric acid was added to the solution while being stirred. The solution was cooled at -20°C for 1 day and the depositing crystals were collected by filtration, to give 3.57g(26%, optical rotation (-) 69.0 (C=1.0, abs. EtOH)) of (*R*)-2-(3' ,4' -dimethoxyphenyl)-2-isopropyl-5-methylaminopentanenitrile di-*p*-toluoyl- L-tartarate.

20ml of water was added to 3.57g(5.3mmol) of the diastereomeric salt and then 4ml of 48% sodium hydroxide solution was further added while being cooled with ice. After being stirred for 10 minutes, the mixture was extracted twice with 50ml of ethyl acetate, to give 1.45g(5.0mmol, 25%) of (*R*)-2-(3' ,4' -dimethoxyphenyl)-2-isopropyl-5-methylamino pentanenitrile.

$^1$H-NMR (d, $CDCl_3$):0.80 (3H, d, *J*=6.5Hz), 1.20 (3H, d, *J*=6.5Hz), 1.14-1.23 (1H, m), 1.54-1.61 (1H, m), 1.86-1.95 (1H, m), 2.08 (1H, sept, *J*=6.5Hz), 2.14-2.22 (1H, m), 2.27 (1H, br-s), 2.37 (3H, s), 2.51-2.61 (2H, m), 3.88 (3H, s), 3.90

(3H, s), 6.83-6.6.87 (2H, m), 6.91-6.95 (1H, m).

Example 20

Synthesis of (*R*)-2-(3' ,4' -dimethoxyphenyl)-2-isopropyl-5-[*N*-methyl-*N*-(3-methylbutyl) amino]-pentanenitrile

1.45g(5.0mmol) of (R)-2-(3 ' ,4' -dimethoxyphenyl)-2-isopropyl-5-methylamino pentanenitrile was dissolved in 5ml of dimethylformamide, and 0.83g(6.0mmol) of potassium carbonate was added thereto. After being stirred at room temperature for 10 minutes, the mixture, with 0.91 g(6.0mmol) of isopentyl bromide added thereto, was stirred at room temperature for 16 hours. The solvent was evaporated out under a vacuum, and the residue was extracted with dichloromethane and washed with water until it became neutral. The organic layer was filtrated through 1PS filter paper and the solvent was evaporated put. The residue was purified by silica gel column chromatography, to give 0.81g (2.2mmol, 44%) of (R)-2-(3' ,4' -dimethoxyphenyl)-2-isopropyl-5-[methyl(3-methylbutyl)amino]-pentanenitrile.
$^1$H-NMR (d, $CDCl_3$):0.80 (3H, d, *J*=6.8Hz), 0.86 (3H, d, *J*=6.8Hz), 0.87 (3H, d, *J*=6.8Hz), 1.10-1.19 (1H, m),1.19 (3H, d, *J*=6.8Hz),1.25-1.32 (2H, m), 1.46-1.57 (2H, m), 1.84 (1H, dt, *J*=4.4,12.2Hz), 2.08 (3H, s), 2.03-2.34 (6H, m), 3.88 (3H, s), 3.89 (3H, s), 6.83-6.87 (2H, m), 6.91-6.95(1H, m).

Example 21

Synthesis of 2-(3' ,4' -dimethoxyphenyl)-2-isopropyl-5-[*N*-methyl-*N*-(3-methylbutyl)amino]-pentanenitrile hydrochloride

1.84g(5.1mmol) of 2-(3 ' ,4' -dimethoxyphenyl)-2-isopropyl-5-[*N*-methyl-*N*-(3-methyl butyl)amino]-pentanenitrile was dissolved in 2ml of dioxane and then 2ml of 4N hydrochloric acid-dioxane solution was added thereto while being cooled with ice. After the mixture was stirred for 5 minutes, the solvent was evaporated out, and then purified by azeotropy with toluene to give 2.02g(5.1mmol, quant.) of 2-(3' ,4' -dimethoxyphenyl)-2-isopropyl-5- *[N*-methyl-*N*-(3-methylbutyl)amino]-pentanenitrile hydrochloride.
$^1$H-NMR (d, DMSO-$d_6$):0.70 (3H, d, *J*=6.8Hz), 0.81-0.87 (6H, m), 1.08-1.20 (3H, m), 1.18-1.32 (1H, m), 1.39-1.65 (4H, m), 2.06-2.26 (3H, m), 2.53-2.60 (3H, m), 2.81-3.13 (4H, m), 3.76 (3H, s), 3.80 (3H, s), 6.93-7.00 (3H, m), 10.44-10.55 (1H, m).

Example 22

Synthesis of*(R)*-2-(3' ,4' -dimethoxyphenyl)-2-isopropyl-5-methylaminopentanenitrilehydrochloride

870mg(3.0mmol) of (*R*)-2-(3' ,4' -dimethoxyphenyl)-2-isopropyl-5-methylamino pentanenitrile was dissolved in 2ml of dioxane, and 1.5ml of 4N hydrochloric acid-dioxane solution was added thereto while being cooled with ice. After the mixture was stirred for 5 minutes, the solvent was evaporated out. The residue obtained by azeotropy with toluene was dissolved in hexane-diethyl ether and placed at -20°C for 1 day. The depositing crystals were collected by filtration, to give 854mg(2.6mmol, 87%) of (*R*)-2-(3',4'-dimethoxy phenyl)-2-isopropyl-5-methylaminopentanenitrile hydrochloride.
$^1$H-NMR (d, DMSO-$d_6$):0.69 (3H, d, *J*=6.3Hz), 1.11 (3H, d, *J*=6.3Hz), 1.13-1.24 (1H, m), 1.49-1.57 (1H, m), 2.09-2.17 (2H, m), 2.20 (1H, sept, *J*=6.3Hz), 2.42 (3H, s), 2.75-2.90 (2H, m), 3.76 (3H, s), 3.79(3H, s), 6.91-7.00 (3H, m), 8.85 (2H, br-s).

Example 23

Synthesis of (*R*)-2-(3',4' -dimethoxyphenyl)-2-isopropyl-5-[*N*-methyl-*N*-(3-methylbutyl) amino]-pentanenitrile hydrochloride

807mg(2.2mmol) of (*R*)-2-(3',4' -dimethoxyphenyl)-2-isopropyl-5-[*N*-methyl-*N*- (3-methylbutyl)amino]-pentanenitrile was dissolved in 2ml of dioxane and 1ml of 4N hydrochloric acid-dioxane solution while being cooled with ice. After the mixture was stirred for 5 minutes, the solvent was evaporated out and then purified by azeotropy with toluene to give 885mg(2.2mmol, quant.) of (*R*)-2-(3' ,4' -dimethoxyphenyl)-2-isopropyl- 5-[*N*-methyl-*N*- (3-methylbutyl) amino]-pentanenitrile hydrochloride.
$^1$H-NMR (d, DMSO-$d_6$):0.70 (3H, d, *J*=6.8Hz), 0.81-0.87 (6H, m), 1.08-1.20 (3H, m), 1.18-1.32 (1H, m), 1.39-1.65 (4H, m), 2.06-2.26 (3H, m), 2.53-2.60 (3H, m), 2.81-3.13 (4H, m), 3.76 (3H, s), 3.80 (3H, s), 6.93-7.00 (3H, m), 10.44-10.55 (1H, m).

Example 24

Synthesis of 2-(3',4' -dimethoxyphenyl)-2-isopropyl-5-morpholine-4"-yl-pentanenitrile

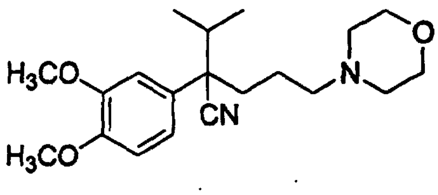

In Example 11, in the place of diethylamine, morpholine was used to give the entitled compound. $^1$H-NMR (d, $CDCl_3$):0.80 (3H, d, *J*=6.8Hz),1.11-1.I9 (1H, m),1.20 (3H, d, *J*=6.8Hz), 1.50-1.61 (1H, m), 1.87 (1H, dt, *J*=4.4,12.9Hz), 2.08 (1H, sept, *J*=6.8Hz), 2.10-2.19 (1H, m), 2.25-2.35 (6H, m), 3.66 (4H, t, *J*=4.8Hz), 3.89(3H, s), 3.89 (3H, s), 6.82-6.86 (2H, m), 6.90-6.93(1H, m).

Example 25

Synthesis of 2-(3',4'-dimethoxyphenyl)-2-isopropyl-5-pyrrole- 1"-yl-pentanenitrile

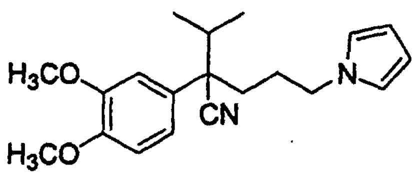

1.00g(3.4mmol) of 5-chloro-2-(3' ,4' -dimethoxyphenyl)-2-isopropylpentanenitrile was dissolved in *N,N*-dimethylformamide, and then 0.62g(5.5mmol) of potassium t-butoxide, 0.37g(5.5mmol) of pyrrole, 0.18g(0.55mmol) of tris(3,6-dioxaheptyl)amine and 0.05g of sodium iodide were added thereto. After the mixture was heated and refluxed for 4 hours, the solvent was evaporated out under a vacuum. The residue was extracted with ethyl acetate and washed with water three times until it became neutral. The organic layer was filtrated through 1PS filter paper and the solvent was evaporated out. The residue was purified by silica gel column chromatography, to give 0.92g(2.8mmol, 83%) of the entitled compound.
$^1$H-NMR (d, $CDCl_3$):0.76 (3H, d, *J*=6.8Hz), 1.15 (3H, d, *J*=6.8Hz), 1.39-1.51 (1H, m), 1.66 (1H, dt, *J*=4.4, 12.8Hz), 1.78-1.89 (1H, m), 2.00 (1H, sept, *J*=6.8Hz), 2.02-2.11 (1H, m), 3.72-3.79 (1H, m), 3.84(3H, s), 3.88 (3H, s), 3.86-3.94 (1H, m), 6.10-6.12 (2H, m), 6.52-6.54 (2H, m), 6.68 (1H, d, *J*=2.0Hz), 6.81-6.88 (2H, m).

Example 26

Synthesis of 2-(3', 4'-dimethoxyphenyl)-5-imidazole-1 "-yl-2-isopropylpentanenitrile

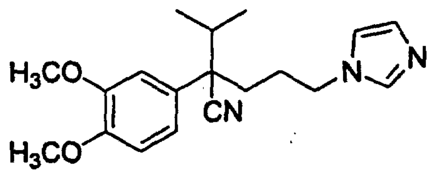

In Example 25, in the place of pyrrole, imidazole was used to give the entitled compound.
$^1$H-NMR (d, $CDCl_3$):0.77 (3H, d, *J*=6.8Hz), 1.16 (3H, d, *J*=6.8Hz), 1.41-1.53 (1H, m), 1.65 (1H, dt, *J*=4.4, 12.8Hz), 1.82-1.93 (1H, m), 2.00 (1H, sept, *J*=6.8Hz), 2.03-2.12 (1H, m), 3.85(3H, s), 3.89 (3H, s), 3.83-3.98 (2H, m), 6.70 (1H, s), 6.77 (1H, s), 6.84 (1H, s), 6.84 (1H, s), 7.05( 1H, s) , 7.39 (1H, m).

Example 27

Synthesis of 2-(3' ,4' -dimethoxyphenyl)-2-isopropyl-5-pyrazole-1"-yl-pentanenitrile

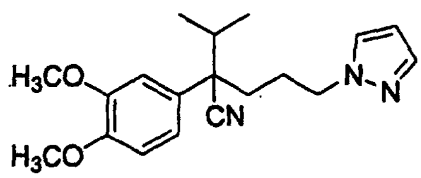

In Example 25, in the place of pyrrole, pyrazole was used to give the entitled compound.
$^1$H-NMR (d, $CDCl_3$):0.77 (3H, d, *J*=6.8Hz), 1.15 (3H, d, *J*=6.8Hz), 1.53-1.65 (1H, m), 1.77 (1H, dt, *J*=4.4, 12.7Hz), 1.84-1.96 (1H, m), 2.01 (1H, sept, *J*=6.8Hz), 2.11 (1H, dt, *J*=4.0, 13.2Hz), 3.85 (3H, s), 3.88(3H, s), 4.06-4.15 (2H, m), 6.23-6.24 (1H, m), 6.71-6.72 (1H, m), 6.81-6.88 (2H, m), 7.28-7.30 (1H, m), 7.45-7.47 (1H, m).

Example 28

Synthesis of 2-(3' ,4' -dimethoxyphenyl)-2-isopropyl-5-[*N*-methyl-*N*-(*n*-pentyl)amino]pentane nitrile

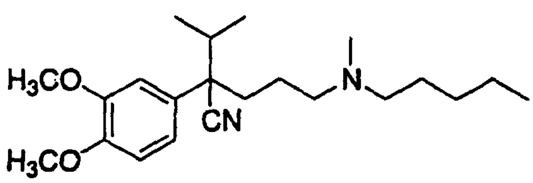

In Example 2, in the place of isopentyl bromide, *n*-pentyl bromide was used to give the entitled compound.
$^1$H-NMR (d, $CDCl_3$):0.80 (3H, d, *J*=6.8Hz), 0.88 (3H, t, *J*=7.3Hz), 1.08-1.44 (7H, m), 1.19 (3H, d, *J*=6.8Hz), 1.47-1.56

(1H, m), 1,84 (1H, dt, $J$=4.4, 12.2Hz), 2.08 (3H, s), 2.04-2.31 (6H, m), 3.88 (3H, s), 3.89 (3H, s), 6.83-6.87 (2H, m), 6.91-6.95 (1H, m).

Further, compounds and manufacturing processes thereof in accordance with the present invention are exemplified.

Examples 29-33

In Example 2, in the place of isopentyl bromide, an alkyl halide shown in Table 5 below can be used to give each compound of Examples 29-33.

Table 5

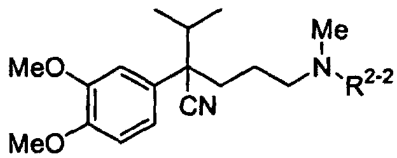

| Example | $R^{2-2}$ | Alkyl halide |
|---|---|---|
| 29 | -*tert*-Bu | *tert*-BuBr |
| 30 | -*sec*-Bu | *sec*-BuBr |
| 31 | -*sec*-Pen | $CH_3CH_2CH_2CH(Br)CH_3$ |
| 32 | -*neo*-Pen | $CH_3C(CH_3)_2CH_2Br$ |
| 33 | -1-Ethylpropyl | $CH_3CH_2CH(Br)CH_2CH_3$ |

Examples 34-46

In Example 1(3), in the place of 30% methylamine methanol solution, ethylamine can be used to give 2-(3' , 4' -dimethoxyphenyl)-5-ethylamino-2-isopropylpentanenitrile

In Example 2, in the place of 2-(3' ,4 -dimethoxyphenyl)-2-isopropyl-5-methylamino pentanenitrile and isopentyl bromide, this compound and an alkyl halide shown in Table 6 below are used respectively, to give each compound of Examples 34-46.

Table 6

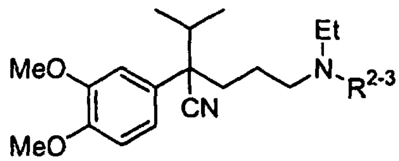

| Example | $R^{2-3}$ | Alkyl halide |
|---|---|---|
| 34 | -*n*-Pr | *n*-PrBr |
| 35 | -*i*-Pr | *i*-PrBr |
| 36 | -*n*-Bu | *n*-BuBr |
| 37 | -*i*-Bu | *i*-BuBr |
| 38 | -*n*-Pen | *n*-PenBr |
| 39 | -*i*-Pen | *i*-PenBr |
| 40 | -EtOEt | EtOEtCl |
| 41 | -EtOEtOMe | MeOEtOEtOMs |
| 42 | -*tert*-Bu | *tert*-BuBr |
| 43 | -sec-Bu | *sec*-BuBr |
| 44 | -*sec*-Pen | $CH_3CH_2CH_2CH(Br)CH_3$ |
| 45 | -*neo*-Pen | $CH_3C(CH_3)_2CH_2Br$ |
| 46 | -1-Ethylpropyl | $CH_3CH_2CH(Br)CH_2CH_3$ |

Examples 47-58

In Example 1(3), in the place of 30% methylamine methanol solution, propylamine is used to give 2-(3' ,4' -dimethoxyphenyl)-2-isopropyl-5-propylaminopentanenitrile.

In Example 2, in the place of 2-(3',4'-dimethoxyphenyl)-2-isopropyl-5-methyl aminopentanenitrile and isopentyl bromide, this compound and an alkyl halide shown in Table 7 below are used respectively, to give each compound of Examples 47-58.

## Table 7

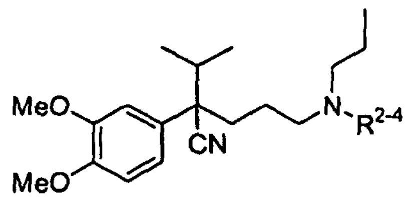

| Example | $R^{2-4}$ | Alkyl halide |
|---|---|---|
| 47 | -*i*-Pr | *i*-PrBr |
| 48 | -*n*-Bu | *n*-BuBr |
| 49 | -*i*-Bu | *i*-BuBr |
| 50 | -*n*-Pen | *n*-PenBr |
| 51 | -*i*-Pen | *i*-PenBr |
| 52 | -EtOEt | EtOEtCl |
| 53 | -EtOEtOMe | MeOEtOEtOMs |
| 54 | -*tert*-Bu | *tert*-BuBr |
| 55 | -*sec*-Bu | *sec*-BuBr |
| 56 | -*sec*-Pen | $CH_3CH_2CH_2CH(Br)CH_3$ |
| 57 | -*neo*-Pen | $CH_3C(CH_3)_2CH_2Br$ |
| 58 | -1-Ethylpropyl | $CH_3CH_2CH(Br)CH_2CH_3$ |

Examples 59-69

In Example 1(3), in the place of 30% methylamine methanol solution, isopropylamine is used to give 2-(3' ,4' -dimethoxyphenyl)-2-isopropyl-5-isopropylamino pentanenitrile.

In Example 2, in the place of 2-(3' ,4' -dimethoxyphenyl)-2-isopropyl-5-methyl aminopentanenitrile and isopentyl bromide, this compound and an alkyl halide shown in Table 8 below are used respectively, to give each compound of Examples 59-69.

# Table 8

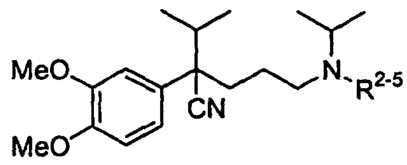

| Example | $R^{2-5}$ | Alkyl halide |
|---|---|---|
| 59 | -*n*-Bu | *n*-BuBr |
| 60 | -*i*-Bu | *i*-BuBr |
| 61 | -*n*-Pen | *n*-PenBr |
| 62 | -*i*-Pen | *i*-PenBr |
| 63 | -EtOEt | EtOEtCl |
| 64 | -EtOEtOMe | MeOEtOEtOMs |
| 65 | -*tert*-Bu | *tert*-BuBr |
| 66 | -*sec*-Bu | sec-BuBr |
| 67 | -*sec*-Pen | $CH_3CH_2CH_2CH(Br)CH_3$ |
| 68 | -*neo*-Pen | $CH_3C(CH_3)_2CH_2Br$ |
| 69 | -1-Ethylpropyl | $CH_3CH_2CH(Br)CH_2CH_3$ |

Examples 70-79

In Example 1(3), in the place of 30% methylamine methanol solution, *n*-butylamine is used to give 5-butylamino-2-(3' ,4' -dimethoxyphenyl)-2-isopropylpentanenitrile.

In Example 2, in the place of 2-(3' ,4' -dimethoxyphenyl)-2-isopropyl-5-methyl aminopentanenitrile and isopentyl bromide, this compound and an alkyl halide shown in Table 9 below are used respectively, to give each compound of Examples 70-79.

## Table 9

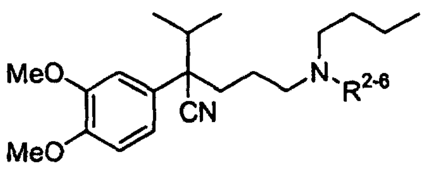

| Example | $R^{2-6}$ | Alkyl halide |
|---|---|---|
| 70 | -*i*-Bu | *i*-BuBr |
| 71 | -*n*-Pen | *n*-PenBr |
| 72 | -*i*-Pen | *i*-PenBr |
| 73 | -EtOEt | EtOEtCl |
| 74 | -EtOEtOMe | MeOEtOEtOMs |
| 75 | -*tert*-Bu | *tert*-BuBr |
| 76 | -*sec*-Bu | *sec*-BuBr |
| 77 | -*sec*-Pen | $CH_3CH_2CH_2CH(Br)CH_3$ |
| 78 | -*neo*-Pen | $CH_3C(CH_3)_2CH_2Br$ |
| 79 | -1-Ethylpropyl | $CH_3CH_2CH(Br)CH_2CH_3$ |

Examples 80-88

In Example 1(3), in the place of 30% methylamine methanol solution, *i*-butylamine is used to give 2-(3',4' -dimethoxyphenyl)-5-isobutylamino-2-isopropylpentanenitrile.

In Example 2, in the place of2-(3' ,4' -dimethoxyphenyl)-2-isopropyl-5-methyl aminopentanenitrile and isopentyl bromide, this compound and an alkyl halide shown in Table 10 below are used respectively, to give each compound of Examples 80-88.

## Table 10

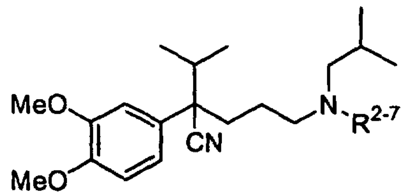

| Example | $R^{2-7}$ | Alkyl halide |
|---|---|---|
| 80 | -*n*-Pen | *n*-PenBr |
| 81 | -*i*-Pen | *i*-PenBr |
| 82 | -EtOEt | EtOEtCl |
| 83 | -EtOEtOMe | MeOEtOEtOMs |
| 84 | -*tert*-Bu | *tert*-BuBr |
| 85 | -*sec*-Bu | *sec*-BuBr |
| 86 | -*sec*-Pen | $CH_3CH_2CH_2CH(Br)CH_3$ |
| 87 | -*neo*-Pen | $CH_3C(CH_3)_2CH_2Br$ |
| 88 | -1-Ethylpropyl | $CH_3CH_2CH(Br)CH_2CH_3$ |

Examples 89-97

In Example 1(3), in the place of 30% methylamine methanol solution, *n*-pentylamine is used to give 2-(3', 4' -dimethoxyphenyl)-2-isopropyl-5-pentylaminopentanenitrile.

In Example 2, using this compound in the place of 2-(3' ,4' -dimethoxyphenyl)- 2-isopropyl-5-methylaminopentanenitrile, the reaction with an alkyl halide shown in Table 11 below is effected in similar manner, to give each compound of Examples 89-97.

Table 11

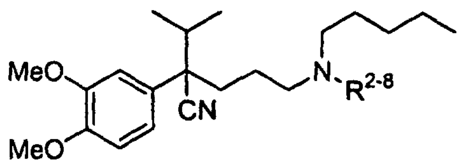

| Example | $R^{2-8}$ | Alkyl halide |
|---|---|---|
| 89 | -*i*-Pen | *i*-PenBr |
| 90 | -*n*-Pen | *n*-PenBr |
| 91 | -EtOEt | EtOEtCl |
| 92 | -EtOEtOMe | MeOEtOEtOMs |
| 93 | -*tert*-Bu | *tert*-BuBr |
| 94 | -*sec*-Bu | *sec*-BuBr |
| 95 | -*sec*-Pen | $CH_3CH_2CH_2CH(Br)CH_3$ |
| 96 | -*neo*-Pen | $CH_3C(CH_3)_2CH_2Br$ |
| 97 | -1-Ethylpropyl | $CH_3CH_2CH(Br)CH_2CH_3$ |

Examples 98-104

In Example 1(3), in the place of 30% methylamine methanol solution, *tert*-butylamine is used to give 5-*tert*-butylamino-2-(3' ,4' -dimethoxyphenyl)-2-isopropyl pentanenitrile.

In Example 2, in the place of 2-(3' ,4' -dimethoxyphenyl)-2-isopropyl-5-methyl aminopentanenitrile and isopentyl bromide, this compound and an alkyl halide shown in Table 12 below are used respectively, to give each compound of Examples 98-104.

# Table 12

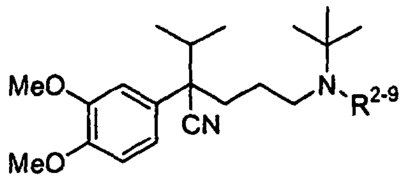

| Example | $R^{2-9}$ | Alkyl halide |
|---|---|---|
| 98 | -*tert*-Bu | *tert*-BuBr |
| 99 | -EtOEt | EtOEtCl |
| 100 | -EtOEtOMe | MeOEtOEtOMs |
| 101 | -*sec*-Bu | *sec*-BuBr |
| 102 | -*sec*-Pen | $CH_3CH_2CH_2CH(Br)CH_3$ |
| 103 | -*neo*-Pen | $CH_3C(CH_3)_2CH_2Br$ |
| 104 | -1-Ethylpropyl | $CH_3CH_2CH(Br)CH_2CH_3$ |

Examples 105-110

In Example 1(3), in the place of 30% methylamine methanol solution, *sec*-butylamine is used to give 5-*sec*-butylamino-2-(3' ,4' -dimethoxyphenyl)-2-isopropyl pentanenitrile.

In Example 2, in the place of 2-(3 ' ,4' -dimethoxyphenyl)-2-isopropyl-5-methyl aminopentanenitrile and isopentyl bromide, this compound and an alkyl halide shown in Table 13 below are used respectively, to give each compound of Examples 105-110.

# Table 13

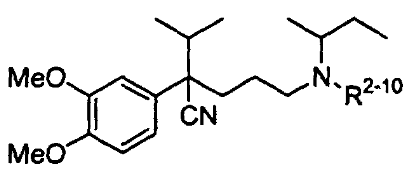

| Example | $R^{2-10}$ | Alkyl halide |
|---|---|---|
| 105 | -*sec*-Bu | *sec*-BuBr |

| | | |
|---|---|---|
| 106 | -EtOEt | EtOEtCl |
| 107 | -EtOEtOMe | MeOEtOEtOMs |
| 108 | -*sec*-Pen | $CH_3CH_2CH_2CH(Br)CH_3$ |
| 109 | -*neo*-Pen | $CH_3C(CH_3)_2CH_2Br$ |
| 110 | -1-Ethylpropyl | $CH_3CH_2CH(Br)CH_2CH_3$ |

Examples 111-115

In Example 1(3), in the place of 30% methylamine methanol solution, *sec*-pentylamine is used to give 2-(3' , 4' -dimethoxyphenyl)-2-isopropyl-5-(1"-methylbutyl amino)pentanenitrile.

In Example 2, in the place of 2-(3' ,4' -dimethoxyphenyl)-2-isopropyl-5-methyl aminopentanenitrile and isopentyl bromide, this compound and an alkyl halide shown in Table 14 below are used respectively, to give each compound of Examples 111-115.

## Table 14

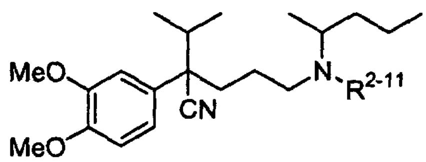

| Example | $R^{2-11}$ | Alkyl halide |
|---|---|---|
| 111 | -sec-Pen | $CH_3CH_2CH_2CH(Br)CH_3$ |
| 112 | -EtOEt | EtOEtCl |
| 113 | -EtOEtOMe | MeOEtOEtOMs |
| 114 | -*neo*-Pen | $CH_3C(CH_3)_2CH_2Br$ |
| 115 | -1-Ethylpropyl | $CH_3CH_2CH(Br)CH_2CH_3$ |

Examples 116-119

In Example 1(3), in the place of 30% methylamine methanol solution, neopentylamine is used to give 2-(3', 4'-dimethoxyphenyl)-5-(2",2"-dimethylpropylamino)- 2-isopropylpentanenitrile.

In Example 2, in the place of 2-(3' ,4' -dimethoxyphenyl)-2-isopropyl-5-methyl aminopentanenitrile and isopentyl bromide, this compound and an alkyl halide shown in Table 15 below are used respectively, to give each compound of Examples 116-119.

# Table 15

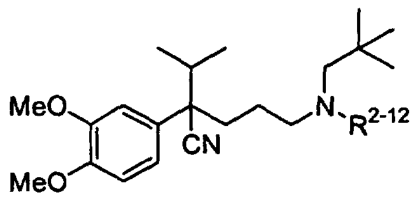

| Example | $R^{2-12}$ | Alkyl halide |
|---|---|---|
| 116 | -*neo*-Pen | $CH_3C(CH_3)_2CH_2Br$ |
| 117 | -EtOEt | EtOEtCl |
| 118 | -EtOEtOMe | MeOEtOEtOMs |
| 119 | -1-Ethylpropyl | $CH_3CH_2CH(Br)CH_2CH_3$ |

Examples 120-122

In Example 1(3), in the place of 30% methylamine methanol solution, 3-aminopentane is used to give 2-(3', 4' -dimethoxyphenyl)-5-(1"-ethylpropylamino)- 2-isopropylpentanenitrile.

In Example 2, in the place of 2-(3' ,4' -dimethoxyphenyl)-2-isopropyl-5-methyl aminopentanenitrile and isopentyl bromide, this compound and an alkyl halide shown in Table 16 below are used respectively, to give each compound of Examples 120-122.

# Table 16

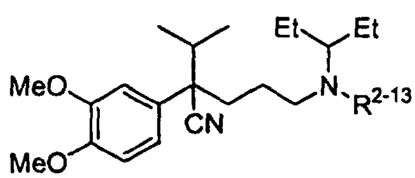

| Example | $R^{2-13}$ | Alkyl halide |
|---|---|---|
| 120 | -1-Ethylpropyl | $CH_3CH_2CH(Br)CH_2CH_3$ |
| 121 | -EtOEt | EtOEtCl |
| 122 | -EtOEtOMe | MeOEtOEtOMs |

In the following, Preparation Examples of the hair growth promoting composition in accordance with the

present invention are shown. The compounding amount is expressed by wt% unless otherwise specified.

| Preparation Example 1 Hair growth tonic | |
|---|---|
| **Example** 21 | 0.5 |
| Pyridoxine dioctanoate | 0.1 |
| Pantothenyl ethyl ether | 0.2 |
| Hinokitiol | 0.05 |
| Polyoxyethylene (12) polyoxypropylene (6) decyl tetradecyl | 1.0 |
| 1-Menthol | 0.1 |
| Disinfectants | Q.S. |
| 1,3-Butylene glycol | 3.0 |
| Ethanol | 70.0 |
| Purified water | Balance |

<Preparation Method>

Ethanol-soluble ingredients were dissolved into ethanol at room temperature while being stirred. Water-soluble ingredients were dissolved in purified water. The aqueous solution was added to the ethanol solution. After being homogeneously mixed, the mixture was filtrated.

| Preparation Example 2 Hair regrowth promoting liquid lotion | |
|---|---|
| Example 22 | 0.2 |
| Carpronium chloride | 1.0 |
| Pantothenyl ethyl ether | 0.5 |
| Diphenhydramine hydrochloride | 0.1 |
| Hinokitiol | 0.1 |
| dl-a-Tocopheryl acetate | 0.1 |
| Salicylic acid | 0.2 |
| 1-Menthol | 0.2 |
| Glycyrrhizinic acid | 0.1 |
| Sodium dl-pyrrolidonecarboxylate solution | 1.0 |
| Ethanol | 70.0 |
| Purified water | Balance |

<Preparation Method>

Ethanol-soluble ingredients were dissolved into ethanol at room temperature while being stirred. Water-soluble ingredients were dissolved in purified water. The aqueous solution was added to the ethanol solution. After being uniformly mixed, the mixture was filtrated.

| Preparation Example 3 Hair growth tonic | |
|---|---|
| Example 23 | 0.5 wt% |
| Pyridoxine dioctanoate | 0.1 |
| Pantothenyl ethyl ether | 0.2 |
| Hinokitiol | 0.05 |
| Polyoxyethylene (12) polyoxypropylene (6) decyl tetradecyl | 1.0 |
| 1-Menthol | 0.1 |
| Disinfectants | Q.S. |
| 1,3-Butylene glycol | 3.0 |
| Ethanol | 70.0 |
| Purified water | Balance |

<Preparation Method>

Ethanol-soluble ingredients were dissolved into ethanol at room temperature while being stirred. Water-soluble ingredients were dissolved in purified water. The aqueous solution was added to the ethanol solution. After being homogeneously mixed, the mixture was filtrated.

| Preparation Example 4 Hair regrowth promoting liquid lotion | |
|---|---|
| Example 21 | 0.2 |
| Carpronium chloride | 1.0 |
| Pantothenyl ethyl ether | 0.5 |
| Diphenhydramine hydrochloride | 0.1 |
| Hinokitiol | 0.1 |
| dl-a-Tocopheryl acetate | 0.1 |
| Salicylic acid | 0.2 |
| 1-Menthol | 0.2 |
| Glycyrrhizinic acid | 0.1 |
| Sodium dl-pyrrolidonecarboxylate solution | 1.0 |
| Ethanol | 70.0 |
| Purified water | Balance |

<Preparation Method>

Ethanol-soluble ingredients were dissolved into ethanol at room temperature while being stirred. Water-soluble ingredients were dissolved in purified water. The aqueous solution was added to the ethanol solution. After being uniformly mixed, the mixture was filtrated.

| Preparation Example 5 O/W milky lotion | |
|---|---|
| (Phase B) | |
| Polyoxyethylene (60) hydrogenated castor oil | 2.0 |
| Glycerin | 10.0 |
| Dipropylene glycol | 10.0 |
| 1,3-Butylene glycol | 4.0 |
| Example 22 | 0.1 |
| Polyethylene glycol 1500 | 5.0 |
| (Phase B) | |
| Isocetyl octanoate | 10.0 |
| Squalane | 5.0 |
| Vaseline | 2.0 |
| Propyl paraben | 2.0 |
| (Phase C) | |
| 1% Carboxyvinylpolymer aqueous solution | 30.0 |
| Sodium hexametaphosphate | 0.03 |
| Ion-exchanged water | 8.35 |
| (Phase D) | |
| Ion-exchanged water | 4.5 |
| (Phase E) | |
| Potassium hydroxide | 0.12 |
| Ion-exchanged water | Balance |

<Preparation Method>

Phases A and B were heated and dissolved, separately. Both were mixed and treated with a homomixer, thereby obtaining a gel. Phase D was then gradually added to this gel and dispersed by a homomixer. Then, Phases C and E, which were mixed and dissolved in advance separately, were added to this gel dispersion successively. The mixture was emulsified by a homomixer to obtain an O/W milky lotion.

| Preparation Example 6 Cream | |
|---|---|
| (Phase A) | |
| *N,N*-Dimethyl-2-tetradecylamineoxide | 2.5 |
| Liquid paraffin | 5.0 |
| Cetostearyl alcohol | 5.5 |
| Glyceryl monostealate | 3.0 |
| Polyoxyethylene (20) 2-octyldodecyl ether | 3.0 |
| Propyl paraben | 0.3 |
| Perfume | 0.1 |
| (Phase B) | |
| Example 21 | 1.0 |
| Glycerin | 8.0 |
| Dipropylene glycol | 20.0 |
| Polyethylene glycol 4000 | 5.0 |
| Sodium hexametaphosphate | 0.005 |
| Ion-exchanged water | Balance |

<Preparation Method>

Phases A and B were heated and dissolved, separately. Both were mixed and emulsified by a homomixer to obtain a cream.

| Preparation Example 7 Aerosol spray | |
|---|---|
| (Stock solution) | |
| 95% Ethanol | 50.0 |
| Glycyrrhizic acid | 0.1 |
| Example 23 | 0.5 |
| Swertia herb extract | 0.1 |
| Sodium lauryl sulfate | 0.1 |
| Polyoxyethylene (40) hydrogenated castor oil | 0.5 |
| Lactic acid | Q.S. |
| Sodium lactate | Q.S. |
| Perfume | Q.S. |
| Ion-exchanged water | Balance |
| (Filling formulation) | |
| Stock solution | 50.0 |
| Liquefied petroleum gas | 50.0 |

<Preparation Method>

A stock solution was prepared by mixing and dissolving the ingredients of stock solution. This stock solution was filled into a can and a valve was fit thereto. The gas was filled into the can to obtain an aerosol spray.

| Preparation Example 8 Shampoo | |
|---|---|
| (1) Sodium cocoylmethyltaurate | 2.0 |
| (2) Polyoxyethylene (8) oleyl ether | 2.0 |
| (3) Lauric acid diethanolamide | 4.0 |
| (4) Ethylene glycol fatty acid ester | 1.0 |
| (5) Glycerin | 0.2 |
| (6) Menthol | 0.1 |
| (7) Example 21 | 0.1 |
| (8) Disodium edetate | 0.1 |
| (9) Perfume | Q.S. |
| (10) Purified water | Balance |

<Preparation Method>

The ingredient (10) was heated up to 70 °C, and the ingredients (1) - (9) were added thereto successively. The mixture was mixed and dissolved with stirring, and then cooled to obtain a shampoo.

| Preparation Example 9 Rinse | |
|---|---|
| (1) Stearyl trimethyl ammonium chloride | 1.5 |
| (2) Dimethyl polysiloxane (20 cs) | 3.0 |
| (3) Polyoxyethylene (10) oleyl ether | 1.0 |
| (4) Glycerin | 5.0 |
| (5) Example 22 | 0.5 |
| (6) 4-*tert*-Butyl-4' -methoxydibenzoylmethane | Q.S. |
| (7) Ultraviolet absorber | Q.S. |
| (8) Purified water | Balance |

<Preparation Method>

The water phase was prepared by adding the ingredients (1), (3) and (4) to the ingredient (8) and heating up to 70°C. The oil phase was prepared by heating and dissolving the other ingredients up to 70°C. The oil phase was added to the water phase and mixed with stirring by an emulsifier. The mixture was cooled to obtain a rinse.

| Preparation Example 10 Scalp treatment | |
|---|---|
| (Stock solution) | |
| (1) Liquid paraffin | 27.0 |
| (2) Stearic acid | 5.0 |
| (3) Cetanol | 5.0 |
| (4) Sorbitan monooleate | 2.0 |
| (5) Polyoxyethylene sorbitan monooleate | 3.0 |
| (6) Example 22 | 0.1 |
| (7) 1,3-Butylene glycol | 5.0 |
| (8) Antiseptic | Q.S. |
| (9) Purified water | Balance |
| (Filling formulation) | |
| Stock solution | 50.0 |
| Liquefied petroleum gas | 50.0 |

<Preparation Method>

The ingredients (5) and (6) were dissolved into the ingredients (1) to (4). After being homogeneously dissolved with heating up to 80°C, the mixture was cooled down to 30°C. This mixture was added to the mixed solution of the ingredients (7) to (9), which was maintained at 30°C, and mixed with stirring to obtain a stock solution. This stock solution was filled into a can together with the propellant to obtain a scalp treatment.

As be explained above, the present invention provides a hair growth promoting composition having excellent effects on promoting hair regrowth, preventing or inhibiting hair loss, and the like in human by compounding specified phenylacetonitrile derivative as a effective ingredient thereto.

## Claims

1. A hair growth promoting composition comprising, as an effective ingredient, a phenylacetonitrile derivative or a pharmacologically acceptable salt thereof expressed by the following Formula (I):

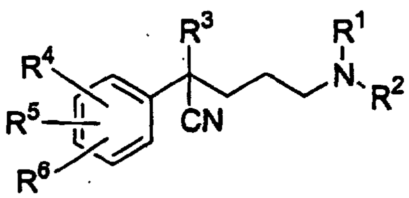

(I)

wherein

each of $R^1$ and $R^2$ is hydrogen atom, a $C_{1-10}$ alkyl group, a $C_{2-10}$ alkenyl group or $C_{2-10}$ acyl group, or $NR^1R^2$ may be a heterocycle having 3-7 members;
$R^3$ is a $C_{1-5}$ alkyl group; and
each of $R^4$, $R^5$ and $R^6$ is hydrogen atom or a $C_{1-4}$ alkoxy group.

2. The hair growth promoting composition according to claim 1, wherein said phenylacetonitrile derivative is expressed by the following Formula (I-1):

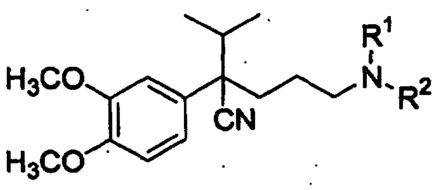

(I-1)

wherein $R^1$ and $R^2$ are as defined in said Formula (I).

3. The hair growth promoting composition according to claim 1 or 2, wherein one of $R^1$ and $R^2$ is hydrogen atom or an alkyl group and the other is an alkyl group.

4. An external preparation for skin comprising a phenylacetonitrile derivative or a pharmacologically acceptable salt thereof expressed by the following Formula (I):

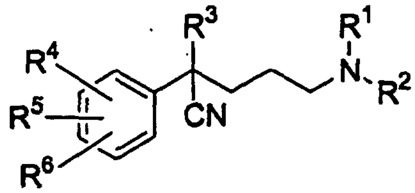

(I)

wherein

each of $R^1$ and $R^2$ is hydrogen atom, a $C_{1-10}$ alkyl group, a $C_{2-10}$ alkenyl group or $C_{2-10}$ acyl group, or $NR^1R^2$ may be a heterocycle having 3-7 members;
$R^3$ is a $C_{1-5}$ alkyl group; and
each of $R^4$, $R^5$ and $R^6$ is hydrogen atom or a $C_{1-4}$ alkoxy group.

**5.** A phenylacetonitrile derivative or a pharmacologically acceptable salt thereof expressed by the following Formula (1-2):

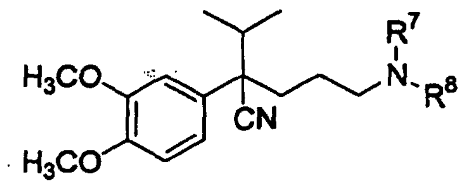

(I-2)

wherein each of $R^7$ and $R^8$ is hydrogen atom, a $C_{1-5}$ alkyl group or a $C_{2-5}$ alkenyl group, or $NR^7R^8$ may be a nonaromatic heterocycle having 3-7 members containing one nitrogen atom or an aromatic heterocycle having 5-7 members containing 1-3 nitrogen atoms, and wherein when one of $R^7$ and $R^8$ is hydrogen atom or methyl group, the other is $C_{2-5}$ alkyl group or $C_{2-5}$ alkenyl group.

## INTERNATIONAL SEARCH REPORT

International application No.
PCT/JP03/03947

A. CLASSIFICATION OF SUBJECT MATTER
Int.Cl$^7$ A61K7/06, A61P17/14, C07C255/42

According to International Patent Classification (IPC) or to both national classification and IPC

B. FIELDS SEARCHED

Minimum documentation searched (classification system followed by classification symbols)
Int.Cl$^7$ A61K07/06, C07C255/42-255/44

Documentation searched other than minimum documentation to the extent that such documents are included in the fields searched

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| X<br>A | EP 434093 A1 (G.D. Searle & Co.),<br>26 June, 1991 (26.06.91),<br>Claim 1<br>& JP 03-291261 A & US 5643947 A | 5<br>1-4 |
| A | US 5059606 A (L'Oreal),<br>22 October, 1991 (22.10.91),<br>Full text<br>& JP 01-172322 A & EP 319983 A1 | 1-5 |
| A | JP 07-238037 A (L'Oreal),<br>12 September, 1995 (12.09.95),<br>Full text<br>& US 6465421 B1 & US 2003/064929 A1<br>& EP 648488 A1 | 1-5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 May, 2003 (21.05.03) | 03 June, 2003 (03.06.03) |
| Name and mailing address of the ISA/<br>Japanese Patent Office | Authorized officer |
| Facsimile No. | Telephone No. |

Form PCT/ISA/210 (second sheet) (July 1998)